(19) Europäisches Patentamt European Patent Office Office européen des brevets

(11) **EP 4 498 202 B1**

(12) **EUROPEAN PATENT SPECIFICATION**

(45) Date of publication and mention of the grant of the patent:
**27.05.2026 Bulletin 2026/22**

(21) Application number: **24189432.8**

(22) Date of filing: **18.07.2024**

(51) International Patent Classification (IPC):
**G05D 23/19** (2006.01)     **G06N 3/045** (2023.01)
**G06N 3/08** (2023.01)

(52) Cooperative Patent Classification (CPC):
**G05D 23/1917; G06N 3/045; G06N 3/08;**
C04B 7/361

(54) **ELECTRONIC DEVICE FOR IMPLEMENTING SYSTEM FOR PREDICTING AND CONTROLLING INDUSTRIAL PROCESSES, AND CONTROL METHOD THEREOF**

ELEKTRONISCHE VORRICHTUNG ZUR IMPLEMENTIERUNG EINES SYSTEMS ZUR VORHERSAGE UND STEUERUNG VON INDUSTRIELLEN PROZESSEN UND STEUERUNGSVERFAHREN DAFÜR

DISPOSITIF ÉLECTRONIQUE POUR METTRE EN UVRE UN SYSTÈME DE PRÉDICTION ET DE COMMANDE DE PROCESSUS INDUSTRIELS, ET SON PROCÉDÉ DE COMMANDE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **25.07.2023 KR 20230096477**

(43) Date of publication of application:
**29.01.2025 Bulletin 2025/05**

(73) Proprietor: **Ineeji Co., Ltd.**
**Seongnam-si, Gyeonggi-do 13558 (KR)**

(72) Inventors:
• **BALJINNYAM, Baasan-Ochir**
**15589 Ansan-si (KR)**
• **LEE, Ye Rim**
**47855 Busan (KR)**
• **YOO, Bo Seon**
**06623 Seoul (KR)**

(74) Representative: **Nederlandsch Octrooibureau**
**P.O. Box 29720**
**2502 LS The Hague (NL)**

(56) References cited:
**WO-A1-2018/038279     WO-A1-2023/067154**
**WO-A1-2023/274992     CN-A- 114 912 712**
**CN-A- 116 382 371**

• **CHEN WEI ET AL: "Model Predictive Control of Outlet Temperature of Calciner Considered RDF Mixing Ratio", 2022 41ST CHINESE CONTROL CONFERENCE (CCC), TECHNICAL COMMITTEE ON CONTROL THEORY, CHINESE ASSOCIATION OF AUTOMATION, 25 July 2022 (2022-07-25), pages 2537 - 2543, XP034203755, DOI: 10.23919/ CCC55666.2022.9902332**

## Description

BACKGROUND

### 1. Field of the Invention

The present disclosure relates to an electronic device and a control method thereof for implementing an industrial process prediction and control system, and more particularly, to an electronic device and a control method thereof for predicting the calorific value of recycled fuel and the temperature of a preheating chamber in a cement manufacturing apparatus using a trained neural network model, and controlling input fuel for cement based on this prediction.

### 2. Discussion of Related Art

In recent industrial processes, technology development is underway to reduce carbon emissions and unnecessary energy expenditure by optimizing indicators such as power and coal consumption, aiming for environmentally friendly operations.

Meanwhile, in the cement manufacturing process, due to the characteristics of the fuel used, the necessity of reducing carbon emissions and unnecessary energy expenditure is further emphasized. The cement process includes a mining process for mining limestone that is a raw material for cement, a crushing process for crushing the mined limestone chunks, a mixing process for mixing the crushed limestone to reduce quality deviation, a raw material grinding process for grinding the mixed limestone into a fine powder along with natural resources (e.g., clay, silica stone, iron ore) or recycled resources (e.g., coal ash, slag, foundry sand, desulfurization gypsum), a preheating process for feeding fine powder raw materials supplied from a raw material storage into the preheating chamber to generate calcium oxide necessary for a firing process, the firing process for heating the generated calcium oxide to a predetermined temperature to produce clinker through a chemical reaction, a cooling process for cooling the produced clinker, and a grinding process for adding gypsum to the cooled clinker and grinding the clinker further to produce the final cement.

In particular, the preheating and the firing processes are carried out in the preheating chamber and kiln included in a cement manufacturing apparatus, respectively. To produce high-quality clinker, the preheating chamber should maintain an appropriate process temperature. When the temperature is lower than the appropriate process temperature, chemical reactions may not occur efficiently. When the temperature is higher than the appropriate process temperature, there is a risk of unnecessary fuel consumption and increased air pollutant gas emissions. Therefore, a method of maintaining the appropriate process temperature is needed.

Meanwhile, to reduce carbon emissions, there is active development of processes using recycled fuel (e.g., waste synthetic resins) in cement production. When using the recycled fuel, there is a problem that the components of the recycled fuel are not uniform and the calorific value changes due to factors such as moisture content. Accordingly, it is difficult to identify the calorific value of the recycled fuel, which makes it difficult to maintain the appropriate process temperature. Patent document WO 2023/274992 A1, published on 5 January 2023, discloses a material feeding control apparatus and method comprising a feed rate measurement device capable of providing an online mass and/or volume flow measurement of material and a material sensing device capable of providing an online content (e.g. composition) measurement of the material. Document CHEN WEI ET AL: "Model Predictive Control of Outlet Temperature of Calciner Considered RDF Mixing Ratio", 2022 41 ST CHINESE CONTROL CONFERENCE (CCC), TECHNICAL COMMITTEE ON CONTROL THEORY, CHINESE ASSOCIATION OF AUTOMATION, pages 2537-2543, published on 25 July 2022, discloses a model predictive controller for the outlet temperature of the calciner considering the RDF mixing ratio. Patent document CN 116 382 371 A, published on 4 July 2023, discloses a decomposition furnace temperature control method comprising the steps that 1, an MISOHammerstein model driven by decomposition furnace data under garbage co-processing is established; and 2, controlling the outlet temperature of the decomposing furnace under the garbage co-processing based on an MISOHammerstein model. Patent document CN 114 912 712 A, published on 16 August 2022, discloses a solid waste co-processing cement batching control method coupled with a feedforward artificial neural network and an evolutionary algorithm, and belongs to the field of cement batching and solid waste treatment recycling. Patent document WO 2023/067154 A1, published on 27 April 2023, discloses the method comprising using an artificial intelligence model and forcasting at least one variable based on an artificial intelligence model, wherein the variable depends on die kiln process. Patent document WO 2018/038279 A1, published on 1 March 2018, discloses a system and a method for predicting temperatures in cement limestone calcination process using neural network learning.

SUMMARY OF THE INVENTION

The present disclosure is directed to providing an electronic device and a control method thereof, which predict the calorific value of recycled fuel using a trained neural network model, predict the temperature of a preheating room using the predicted calorific value of the recycled fuel, and provide guidance information to a user to maintain an appropriate process temperature based on the predicted temperature.

According to an aspect of the present disclosure, there is provided an electronic device for implementing a temperature prediction and control system, which includes a communication interface, a memory in which a trained first neural network model and a trained second neural network model are stored, and one or more processors configured to perform preprocessing on, when process information including fuel input information of a cement manufacturing apparatus is received through the communication interface, the received process information.

The one or more processors may input the preprocessed process information into the trained second neural network model to obtain error information on first predicted temperature information output from the trained first neural network model and the measured temperature of a first preheating chamber in the cement manufacturing apparatus.

The one or more processors may identify predicted calorific information of a first recycled fuel among input fuels based on the obtained error information and the fuel input information.

The one or more processors may input the fuel input information updated based on the identified predicted calorific information into the trained first neural network model to obtain second predicted temperature information of the first preheating chamber.

The one or more processors may provide guidance information including the obtained second predicted temperature information.

According to another aspect of the present disclosure, there is provided a control method of an electronic device for implementing a temperature prediction and control system, which includes performing preprocessing on, when process information including fuel input information of a cement manufacturing apparatus is received, the received process information.

The control method may include inputting the preprocessed process information into a trained second neural network model to obtain error information on first predicted temperature information output from a trained first neural network model and the measured temperature of a first preheating chamber in the cement manufacturing apparatus.

The control method may further include identifying predicted calorific information of a first recycled fuel among the input fuels based on the obtained error information and the fuel input information.

The control method may further include inputting the fuel input information updated based on the identified predicted calorific information into the trained first neural network model to obtain second predicted temperature information of the first preheating chamber.

The control method may further include providing guidance information including the obtained second predicted temperature information.

According to still another aspect of the present disclosure, there is provided a non-transitory computer-readable recording medium that stores, when executed by a processor of an electronic device for implementing a temperature prediction and control system, a computer command that causes the electronic device to perform operations of performing preprocessing, when process information including fuel input information of a cement manufacturing apparatus is received, on the received process information.

The operation may include inputting the preprocessed process information into a trained second neural network model to obtain error information on first predicted temperature information output from a trained first neural network model and the measured temperature of a first preheating chamber in the cement manufacturing apparatus.

The operation may include identifying predicted calorific information of a first recycled fuel among input fuels based on the obtained error information and the fuel input information.

The operation may include inputting the fuel input information updated based on the identified predicted calorific information into the trained first neural network model to obtain second predicted temperature information of the first preheating chamber.

The operation may include providing guidance information including the obtained second predicted temperature information.

BRIEF DESCRIPTION OF THE DRAWINGS

The above and other objects, features and advantages of the present disclosure will become more apparent to those of ordinary skill in the art by describing exemplary embodiments thereof in detail with reference to the accompanying drawings.

FIG. 1 is a diagram schematically illustrating a control method of an electronic device according to one embodiment.

FIG. 2 is a block diagram illustrating a configuration of an electronic device according to one embodiment.

FIG. 3 is a flowchart illustrating a control method of an electronic device according to one embodiment.

FIG. 4 is a diagram illustrating a trained first neural network model according to one embodiment, and a training method of the trained first neural network model.

FIG. 5 is a diagram illustrating a trained second neural network model according to one embodiment, and a training method of the trained second neural network model.

FIG. 6 is a diagram illustrating a method of providing guidance information according to one embodiment.

FIG. 7 is a diagram illustrating a method of obtaining and providing guidance information according to one embodiment.

FIGS. 8A and 8B are diagrams illustrating a method of providing a user interface (UI) according to one embodiment.

FIGS. 9A, 9B, and 9C are diagrams illustrating a method of providing a UI according to one embodiment.

FIG. 10 is a diagram illustrating a method of identifying control information and transmitting the identified control information to a control engine according to one embodiment.

FIG. 11 is a block diagram illustrating a detailed configuration of an electronic device according to one embodiment.

DETAILED DESCRIPTION OF EXEMPLARY EMBODIMENTS

Hereinafter, the present disclosure will be described in detail with reference to the accompanying drawings.

Terms used in this specification will be briefly described, and then the present disclosure will be described in detail.

The terms used in the embodiments of the present disclosure have selected general terms that are currently widely used as much as possible while considering the functions in the present disclosure, but this may vary depending on the intention or precedent of a technician working in the art, the emergence of new technology, etc. In addition, in certain cases, there are terms arbitrarily selected by the applicant, and in this case, the meaning will be described in detail in the description part of the relevant disclosure. Therefore, the terms used in this disclosure should be defined based on the meaning of the term and the overall content of this disclosure, rather than simply the name of the term.

In this specification, expressions such as "have." "may have," "include," and "may include" refer to the presence of the corresponding feature (e.g., a component such as a numerical value, a function, an operation, or a part), and does not rule out the existence of additional features.

The expression at least one of A or/and B should be understood as referring to either "A" or "B" or "A and B."

As used herein, expressions such as "first," "second," "first," and "second," may be used for various components regardless of order and/or importance, and it is only used to distinguish one component from other components and does not limit the components.

When it is mentioned that any component (e.g., a first component) is (operatively or communicatively) coupled with/to or is connected to another component (e.g., a second component), it is to be understood that any component is directly coupled with/to another component or may be coupled with/to another component through still another component (e.g., a third component).

Singular expressions include plural expressions unless the context clearly dictates otherwise. In this application, terms such as "comprise" and "consist of" are intended to designate the presence of features, numbers, steps, operations, components, parts, or combinations thereof described in the specification, but do not preclude the possibility of the presence or addition of one or more other features, numbers, steps, operations, components, parts, or combinations thereof.

In the embodiments, a "module" or a "unit" may perform at least one function or operation, and be implemented as hardware or software or be implemented as a combination of hardware and software. In addition, a plurality of "modules" or a plurality of "units" may be integrated in at least one module and be implemented as at least one processor (not shown) except for a "module" or a "unit" that needs to be implemented as specific hardware.

An electronic device according to one embodiment of the present disclosure may include an artificial intelligence model (or artificial neural network model or learning network model) composed of at least one neural network layer. The artificial neural network may include deep neural network (DNN), such as convolutional neural network (CNN), recurrent neural network (RNN), restricted Boltzmann machine (RBM), deep belief network (DBN), bidirectional recurrent deep neural network (BRDNN) or deep Q-network, etc., but are not limited to the above examples.

In addition, in this specification, "parameter" is a value used in the calculation process of each layer constituting a neural network and may include, for example, a weight used when applying an input value to a predetermined calculation equation. In addition, parameters can be expressed in matrix form. Parameters are values set as a result of training, and can be updated through separate training data as needed.

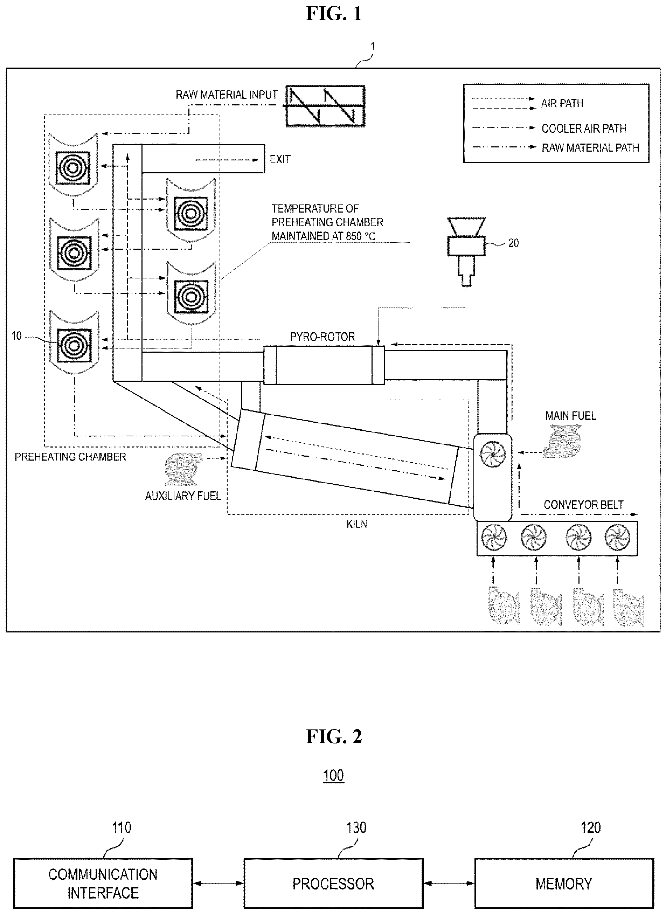

FIG. 1 is a diagram schematically illustrating a control method of an electronic device according to one embodiment.

Referring to FIG. 1, first, a firing process is performed in a kiln included in a cement manufacturing apparatus 1 according to one embodiment. In order to perform the cement process successfully, the temperature of a kiln in which the firing process is performed should be within a predetermined range (1500 °C to 2000 °C according to one embodiment). The cement manufacturing apparatus 1 may include a plurality of preheating chambers. Among these, the temperature of a first preheating chamber 10 connected to the pyro-rotor and the kiln should also be within a predetermined range to ensure that efficient processing can be performed.

Meanwhile, the cement manufacturing apparatus 1 may include a plurality of fuel inlets. According to one embodiment, the cement manufacturing apparatus 1 may include an auxiliary fuel inlet, a main fuel inlet, and a recycled fuel inlet 20. By introducing recycled fuel into the cement manufacturing apparatus 1, carbon emissions are reduced and energy consumption decreases. However, due to the characteristics of the recycled fuel, it is difficult to measure the exact calorific value, which makes it difficult to control the temperature of the preheating chamber and kiln.

Hereinafter, various embodiments in which the calorific value of the recycled fuel input into the cement manufacturing device is predicted, the temperature of the first preheating chamber 10 connected to the pyro-rotor and the kiln is predicted based on the predicted calorific value, and guidance information for increasing the ratio of the recycled fuel while maintaining the temperature of the first preheating chamber within a predetermined range is provided will be described.

FIG. 2 is a block diagram illustrating a configuration of an electronic device according to one embodiment.

Referring to FIG. 2, the electronic device 100 may include a communication interface 110, a memory 120, and one or more processors 130.

The electronic device 100 according to one embodiment may be implemented as a device that processes data and communicates with an external device, such as a server, but is not limited to this. For example, the electronic device 100 may be implemented as various devices such as a smart TV, tablet, monitor, smartphone, desktop computer, laptop computer, etc. The electronic device 100 according to one embodiment of the present disclosure is not limited to the above-mentioned devices, and the electronic device 100 may be implemented as an electronic device 100 having two or more functions of the above-mentioned devices.

Meanwhile, the electronic device 100 may communicate with external devices and external servers in various ways. According to one embodiment, communication modules for communication with external devices and external servers may be implemented in the same way. For example, the electronic device 100 may communicate with an external device using a Bluetooth module, and also communicate with an external server using the Bluetooth module.

According to another embodiment, communication modules for communication with external devices and external servers may be implemented separately. For example, the electronic device 100 may communicate with an external device using a Bluetooth module and communicate with an external server using an Ethernet modem or a Wi-Fi module.

Meanwhile, according to one embodiment, an external device (not shown) may be implemented as a cement manufacturing apparatus, but is not limited thereto.

The communication interface 110 may transmit and receive various types of data to and from external devices (e.g., source devices), external storage media (e.g., USB memory), and external servers (e.g., web hard drives) through a communication method such as AP-based Wi-Fi (a wireless local area network (LAN) network), Bluetooth, ZigBee, a wired/wireless LAN, a wide area network (WAN), Ethernet, IEEE 1394, a high-definition multimedia interface (HDMI), a Universal Serial Bus (USB), a mobile high-definition link (MHL), audio an engineering society/European broadcasting union (AES/EBU), an optical method, or a coaxial method.

According to one embodiment, the communication interface 110 may include a Bluetooth low energy (BLE) module. BLE refers to a Bluetooth technology that enables transmission and reception of low-power and low-capacity data in a frequency band of 2.4GHz with a reach radius of approximately 10m. However, the communication interface 110 is not limited to the BLE module, and include a Wi-Fi communication module. That is, the communication interface 110 may include at least one of a BLE module or a Wi-Fi communication module.

According to one embodiment, the communication interface 110 may use a different communication module to communicate with an external device and an external server, such as a remote control device. For example, the communication interface 110 may use at least one of an Ethernet module or a Wi-Fi module to communicate with an external server, and may use a Bluetooth module to communicate with an external device such as a remote control device. However, this is only an example, and the communication interface 110 may use at least one communication module among various communication modules when communicating with a plurality of external devices or external servers.

The memory 120 may store data needed for various embodiments. The memory 120 may be implemented as a memory embedded in the electronic device 100 or as a memory detachable from the electronic device 100 depending on the data storage purpose. For example, data for driving the electronic device 100 may be stored in the memory embedded in the electronic device 100, and data for the expansion function of the electronic device 100 may be stored in the memory detachable from the electronic device 100.

Meanwhile, in the case of the memory embedded in the electronic device 100, it may be implemented as at least one of a volatile memory (e.g., a dynamic RAM (DRAM), a static RAM (SRAM), or a synchronous dynamic RAM (SDRAM)), and a non-volatile memory (e.g., a one time programmable ROM (OTPROM), a programmable RAM (PROM), an erasable and programmable ROM (EPROM), an electrically erasable and programmable ROM (EEPROM), a mask ROM, a flash ROM, a flash memory (e.g., a NAND flash, a NOR flash, etc.), a hard drive, or a solid state drive (SSD)). In addition, in the case of the memory detachable from the electronic device 100, it may be implemented in the form of a memory card (e.g., a compact flash (CF) card, a secure digital (SD) card, a micro secure digital (Micro-SD) card, a mini secure digital (Mini-SD) card, an extreme digital (xD) card, a multi-media card (MMC), etc.), an external memory connectable to a USB port, or the like.

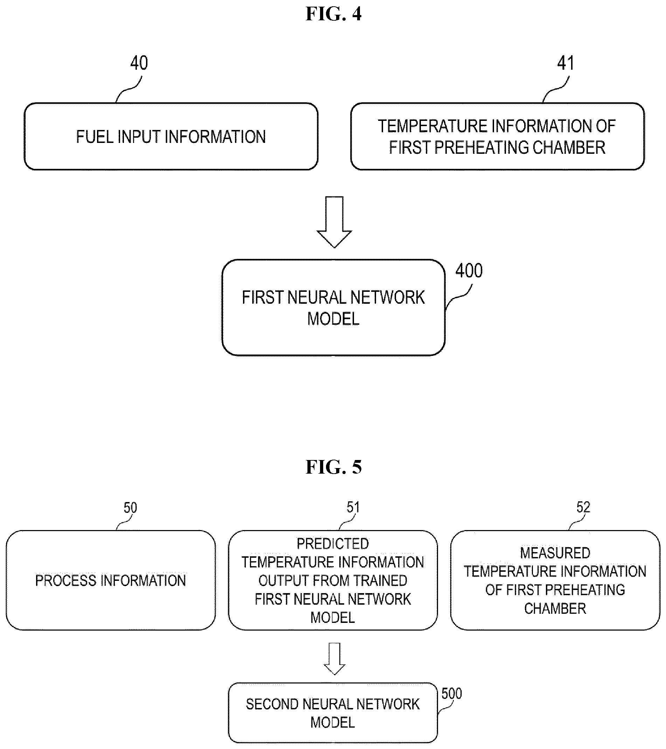

The memory 120 stores a trained first neural network model and a trained second neural network model. Meanwhile, the trained first neural network model and the trained second neural network model will be described in detail with reference to FIGS. 4 and 5.

The one or more processors 130 (hereinafter referred to as processor) are electrically connected to the communication interface 110 and the memory 120 to control the overall operation of the electronic device 100. The processor 130 may be comprised of one or multiple processors. Specifically, the processor 130 may perform the operation of the electronic device 100 according to various embodiments of the present disclosure by executing at least one instruction stored in the memory 120.

According to one embodiment, the processor 130 may be implemented as a digital signal processor (DSP), a microprocessor, a graphics processing unit (GPU), an artificial intelligence (AI) processor, a neural processing unit (NPU), or a time controller (TCON), but is not limited thereto. The processor 130 may include one or more of a central processing unit (CPU), a micro controller unit (MCU), a micro processing unit (MPU), a controller, an application processor (AP), a communication processor (CP), and an ARM processor, or may be defined by the corresponding term. In addition, the processor 130 may be implemented as a System on Chip (SoC) with a built-in processing algorithm, a large scale integration (LSI), or as an application specific integrated circuit (ASIC) or a field programmable gate array (FPGA).

According to one embodiment, the processor 130 may be implemented as a DSP, a microprocessor, or a TCON. However, the processor 130 is not limited thereto, and may include one or more of a CPU, an MCU, an MPU, a controller, an AP, a CP, and an ARM processor, or may be defined by the corresponding term. In addition, the processor 130 may be implemented as a SoC or an LSI with a built-in processing algorithm, or may be implemented in the form of an FPGA.

According to one embodiment, the processor 130 may receive process information including fuel input information of the cement manufacturing apparatus 1 through the communication interface 110. Here, the process information is information on the overall environment of the cement manufacturing apparatus 1 in which cement processing is performed. According to one embodiment, the process information may include fuel input information on fuel input into the cement manufacturing apparatus 1 for cement production or process state information corresponding to the cement manufacturing apparatus 1.

According to one example, the fuel input information may include information on the input amount corresponding to each of the plurality of types of fuel input for cement production and calorific information corresponding to each of the plurality of types of fuel. The plurality of types of fuel may include, for example, bituminous coal-type main fuel, bituminous coal-type auxiliary fuel, and a plurality of types of recycled fuel. Here, the main fuel and the auxiliary fuel are the same type of fuel, but the main fuel is supplied through a first fuel input unit located on one side of the kiln within the cement manufacturing apparatus 1, and the auxiliary fuel is supplied through a second fuel input unit located on the other side of the kiln. The auxiliary fuel is fuel for supplementing the main fuel.

Meanwhile, the recycled fuel is fuel used for eco-friendly processes and carbon emission reduction, and has a relatively low carbon content compared to the bituminous coal-type main fuel and auxiliary fuel, thereby reducing carbon emissions and reducing unnecessary energy expenditure. According to one embodiment, the recycled fuel may include at least one of waste synthetic resin-type fuel, waste tire-type fuel, waste oil-type fuel, and sewage sludge-type fuel.

Meanwhile, the process state information corresponding to the cement manufacturing apparatus 1 may include, for example, process environment information for the cement manufacturing apparatus 1. According to one example, the process state information may include at least one of the oxygen concentration, carbon dioxide concentration, and nitrogen oxide concentration in the exhaust gas discharged from the inlet of the kiln in the cement manufacturing apparatus 1. Alternatively, the process state information may include the temperature of the air input to a pyro-rotor and kiln after a cooling process, pressure information of a fan within a cooler, speed information of a conveyor belt, speed information and current amount information of the pyro-rotor and kiln, and information on the raw materials (e.g., lime powder) input to the cement manufacturing apparatus 1, but is not limited thereto.

According to one embodiment, the processor 130 may receive process information including the fuel input information of the cement manufacturing apparatus 1 from an external server (not shown) that stores the process information of the cement manufacturing apparatus 1 through the communication interface 110. Alternatively, the processor 130 may receive process information from a sensor provided in the cement manufacturing apparatus 1 through the communication interface 110, but is not limited thereto.

When the process information is received, the processor 130 performs preprocessing on the received process information. According to one embodiment, the processor 130 may identify outlier data among a plurality of pieces of data included in the received process information using a predetermined algorithm, and obtain process information from which the identified outlier data has been removed. Alternatively, when the process information corresponding to a predetermined time period is not received during the time the process is performed, the processor 130 may perform preprocessing by receiving the process information that has not been received from the communication interface 110. However, the present disclosure is not limited to this.

According to one embodiment, the processor 130 may input the preprocessed process information into a trained second neural network model to obtain error information on first predicted temperature information output from a trained first neural network model and the measured temperature of a first preheating chamber in the cement manufacturing apparatus 1.

According to one embodiment, when the process information including the fuel input information and the process state information is input, the trained second neural network model may be a neural network model trained to output error information on predicted temperature information output from the trained first neural network model and measured temperature information of the first preheating chamber. The processor 130 may obtain the error information by inputting the preprocessed process information including the fuel input information and the process state information into the trained second neural network model.

Here, the error information on the predicted temperature information output from the trained first neural network model and the measured temperature information of the first preheating chamber refers to an error caused by a change in the calorific value of a waste synthetic resin among a plurality of recycled fuel types. That is, in the case of the waste synthetic resin, it is difficult to accurately identify the calorific value due to the characteristics of the fuel, and as the amount of heat of the waste synthetic resin is not accurately identified, an error occurs in the predicted temperature value and the measured temperature value within the first preheating chamber. According to this disclosure, it is possible to accurately identify the calorific value of the waste synthetic resin among recycled fuels to precisely predict the temperature of the first preheating chamber.

Meanwhile, in one embodiment, the plurality of types of temperature information, including the first predicted temperature information and the second predicted temperature information, may include temperature value information.

The processor 130 identifies the predicted calorific information of first recycled fuel among the input fuels based on the obtained error information and the fuel input information. Here, the first recycled fuel may be a waste synthetic resin, and the processor 130 may identify the predicted calorific information of the first recycled fuel based on the obtained error information and information on the input amount of the first recycled fuel included in the fuel input information. For example, the processor 130 may identify the predicted calorific value of the waste synthetic resin using the following Equation 1.

[Equation 1]

$$A = B + \frac{C}{D}$$

Here, A denotes a predicted calorific value of the waste synthetic resin, B denotes the existing calorific value of the waste synthetic resin used to predict the temperature of the first preheating chamber, C denotes error information for the temperature of the first preheating chamber output from the trained second neural network model, and D denotes the input amount of the waste synthetic resin. According to one embodiment, it is assumed that the calorific value of the existing waste synthetic resin is 10, the error size is 50, and the input amount is 100. The processor 130 may identify the predicted calorific value of the waste synthetic resin as 10.5 (10+(50/100)) through Equation 1. This allows the processor 130 to accurately identify the calorific value of the waste synthetic resin.

According to one embodiment, the processor 130 may obtain second predicted temperature information of the first preheating chamber by inputting fuel input information updated based on the identified predicted calorific information into the trained first neural network model. According to one embodiment, the processor 130 may input, into the trained first neural network model, the fuel input information in which the identified predicted calorific information of the waste synthetic resin is updated, and obtain the second predicted temperature information of the first preheating chamber from the trained first neural network model. Here, the first predicted temperature information is predicted temperature information of the first preheating chamber corresponding to the fuel input information before the calorific information of the first recycled fuel, i.e., the waste synthetic resin is updated, and the second predicted temperature information is predicted temperature information of the first preheating chamber corresponding to the fuel input information after the calorific information of the first recycled fuel, i.e., the waste synthetic resin is updated.

The processor 130 provides guidance information including the obtained second predicted temperature information. According to one embodiment, when the obtained second predicted temperature information is identified as exceeding a target temperature value, the processor 130 may obtain guidance information to ensure that the input amount of at least one of the main fuel and the auxiliary fuel is reduced. Alternatively, according to one embodiment, when the obtained second predicted temperature information is identified as being less than the target temperature value, the processor 130 may obtain guidance information to ensure that the input amount of the first recycled fuel is increased.

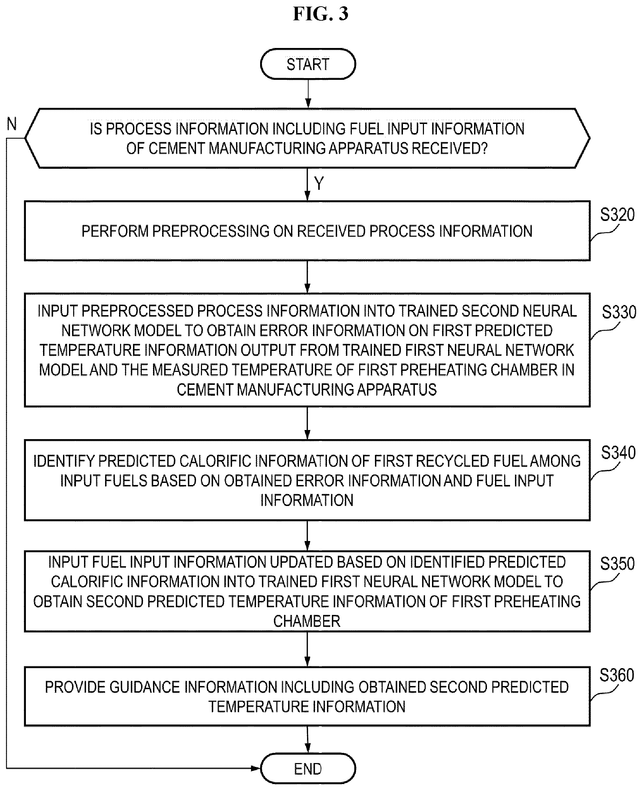

FIG. 3 is a flowchart illustrating a control method of an electronic device according to one embodiment.

According to one embodiment, first, when process information including fuel input information of the cement manufacturing apparatus 1 is received (Y in S310), in operation S320, the control method may perform preprocessing on the received process information. According to one example, the processor 130 may identify outlier data among a plurality of pieces of data included in the received process information using a predetermined algorithm, and obtain the process information from which the identified outlier data has been removed.

Next, according to one embodiment, in operation S330, the control method may input the preprocessed process information to a trained second neural network model to obtain error information on first predicted temperature information output from a trained first neural network model and the measured temperature of a first preheating chamber in the cement manufacturing apparatus 1.

Next, according to one embodiment, in operation S340, the control method may identify predicted calorific information of a first recycled fuel among the input fuels based on the obtained error information and fuel input information. According to one example, the processor 130 may identify error information on the temperature of the first preheating chamber output from the trained second neural network model, information on the input amount of the first recycled fuel corresponding to a waste synthetic resin among a plurality of types of input fuel, and predicted calorific information of the first recycled fuel based on calorific information on the existing waste synthetic resin.

Next, according to one embodiment, in operation S350, the control method may input fuel input information updated based on the identified predicted calorific information into the trained first neural network model to obtain second predicted temperature information of the first preheating chamber. According to one embodiment, the processor 130 may update the fuel input information based on the identified predicted calorific information using Equation 1, and input the updated fuel input information into the trained first neural network model to obtain the second predicted temperature information of the first preheating chamber.

Next, according to one embodiment, in operation S360, the control method may provide guidance information including the obtained second predicted temperature information. According to one embodiment, when the obtained second predicted temperature information is identified as exceeding a target temperature value, the processor 130 may obtain guidance information to ensure that the input amount of at least one of main fuel and auxiliary fuel is reduced. Alternatively, according to one embodiment, when the obtained second predicted temperature information is identified as being less than the target temperature value, the processor 130 may obtain guidance information to ensure that the input amount of the first recycled fuel is increased. The processor 130 may provide the obtained guidance information.

tion.

However, the present disclosure is not limited thereto, and according to one embodiment, the processor 130 may provide guidance information on input amounts for different types of fuel other than the main fuel, the auxiliary fuel, and the recycled fuel. Alternatively, according to one embodiment, the processor 130 may also provide guidance information on the pressure of a cooler fan included in the cement manufacturing apparatus 1.

According to the above-described example, the electronic device 100 may predict the calorific value of the recycled fuel using the trained neural network model, and predict the temperature of the first preheating chamber based on the predicted calorific value. Accordingly, the electronic device 100 may provide the guidance information to the user based on the accurately predicted temperature of the preheating chamber, thereby enabling an eco-friendly and efficient process.

FIG. 4 is a diagram illustrating a trained first neural network model according to one embodiment, and a training method of the trained first neural network model.

An artificial neural network (or neural network model) including a trained first neural network model and a trained second neural network model according to one embodiment of the present disclosure may include a DNN. For example, the artificial neural network may include a CNN, an RNN, an RBM, a DBN, a BRDNN, deep Q-networks, etc., but is not limited to the above examples.

According to one embodiment, the trained first neural network model may be stored in the memory 120. When information on the input amount corresponding to each of different types of input fuels including the first recycled fuel and the fuel input information including calorific information corresponding to each of the input fuels are input, the trained first neural network model may be trained to output predicted temperature information of the first preheating chamber.

Referring to FIG. 4, according to one embodiment, a data set containing fuel input information 40 including the input amount information and calorific information of fuel corresponding to each of the main fuel, auxiliary fuel, and a plurality of types of recycled fuel may be input into a first neural network model 400 as training data to allow the neural network model to be trained. In this case, the data set may include the temperature information 41 of the first preheating chamber of the first preheating chamber, which is measured based on the fuel input, as a label.

For example, a data set containing the input amount information and calorific information of fuel corresponding to each of the main fuel, auxiliary fuel, and a plurality of types of recycled fuel at an $n^{th}$ time point may be input into the first neural network model as training data to allow the neural network model to be trained. In this case, the data set may include the temperature information of the first preheating chamber at an $(n+1)^{th}$ time point of the first preheating chamber, which is measured based on the fuel input at the $n^{th}$ time point, as a label to allow training to be performed.

FIG. 5 is a diagram illustrating a trained second neural network model according to one embodiment and a training method of the trained second neural network model.

According to one embodiment, a trained second neural network model may be stored in the memory 120. According to one embodiment, when process information including fuel input information and process state information is input, the trained second neural network model may be trained to output error information on predicted temperature information output from the trained first neural network model and measured temperature information of the first preheating chamber. Here, the measured temperature information of the first preheating chamber is information on the first preheating chamber measured through an actual sensor.

Referring to FIG. 5, a data set containing process information 50 including the fuel input information and the process state information input into the trained second neural network model 500 as training data to allow the second neural network model 500 to be trained. Here, the fuel input information may include the input amount information and calorific information of fuel corresponding to each of main fuel, auxiliary fuel, and a plurality of types of recycled fuel. The process state information includes process environment information on the cement manufacturing apparatus 1. According to one embodiment, the process state information may include at least one of the oxygen concentration, carbon dioxide concentration, and nitrogen oxide concentration in the exhaust gas discharged from the inlet of the kiln within the cement manufacturing apparatus 1. Alternatively, the process state information may include the temperature of the air input to a pyro-rotor and kiln after a cooling process, pressure information of a fan within a cooler, speed information of a conveyor belt, speed information and current amount information of the pyro-rotor and kiln, and information on the raw materials (e.g., lime powder) input to the cement manufacturing apparatus 1, but is not limited thereto.

Meanwhile, the data set may include a difference value between predicted temperature information 51 of the first preheating chamber output from the trained first neural network model and measured temperature information 52 of the first preheating chamber as a label. That is, the second neural network model 500 may perform training using the predicted temperature information output from the trained first neural network model as training data. Meanwhile, according to one embodiment, the process information may include temperature history information of the first preheating chamber and history information of the input fuel. The measured temperature information 52 of the first preheating chamber may be obtained based on the temperature history information of the first preheating chamber included in the process information.

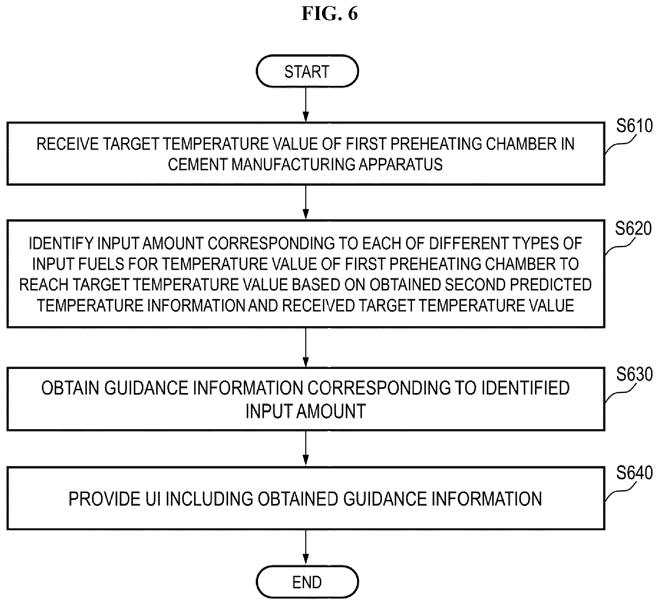

FIG. 6 is a diagram illustrating a method of providing guidance information according to one embodiment.

Referring to FIG. 6, according to one embodiment, first, in operation S610, the control method may receive a target temperature value of the first preheating chamber in the cement manufacturing apparatus through a user interface (not shown).

Next, according to one embodiment, the control method may identify the input amount corresponding to each of different types of input fuels for the temperature value of the first preheating chamber to reach a target temperature value in operation S620, and obtain guidance information corresponding to the identified input amount in operation S630.

According to one example, the memory 120 may previously store information on a change value of the temperature of the first preheating chamber according to a change in the unit input amount corresponding to each of a plurality of types of input fuel including main fuel and auxiliary fuel. According to one embodiment, the processor 130 may obtain information on the change value of the temperature of the first preheating chamber according to the change in the unit input amount through the process information including process history information using a predetermined algorithm. The processor 130 may identify the input amount of the main fuel or auxiliary fuel to be input based on the information stored in the memory 120 and a difference value between the second predicted temperature information and the target temperature value, and obtain guidance information including information on the identified input amount. Alternatively, the processor 130 may identify the input amount of a first recycled fuel based on the information stored in the memory 120 and a difference value between the second predicted temperature information and the target temperature value, and obtain guidance information including information on the identified input amount.

For example, when the second predicted temperature information exceeds the target temperature value, the processor 130 may obtain guidance information to ensure that the input amount of at least one of the bituminous coal-type main fuel and auxiliary fuel is changed to allow the temperature value of the first preheating chamber to reach the target temperature value. Alternatively, for example, when the second predicted temperature information is less than the target temperature value, the processor 130 may obtain guidance information to ensure that the input amount of the first recycled fuel is increased. When it is identified that the second predicted temperature information is less than the target temperature value based on the information stored in the memory 120, the processor 130 may obtain guidance information to ensure that the input amount of a waste synthetic resin corresponding to the first recycled fuel is increased.

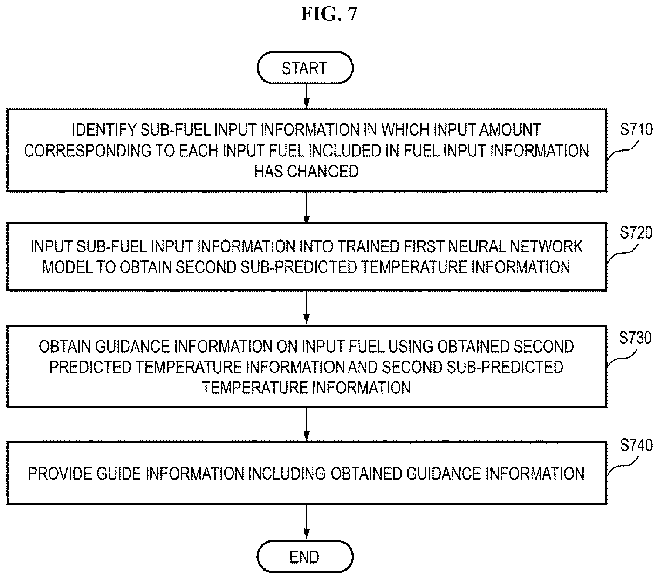

However, the present disclosure is not limited thereto, and the processor 130 may obtain guidance information corresponding to each magnitude of predicted temperature information using the trained first and second neural network models. This will be described in detail with reference to FIG. 7.

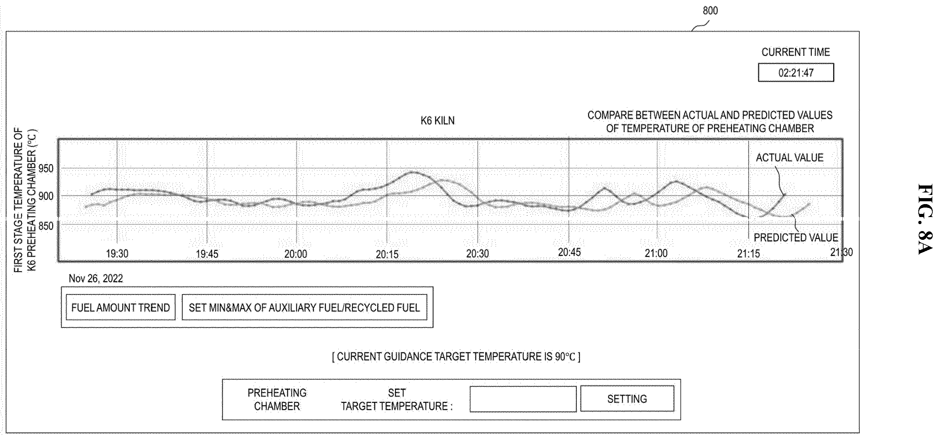

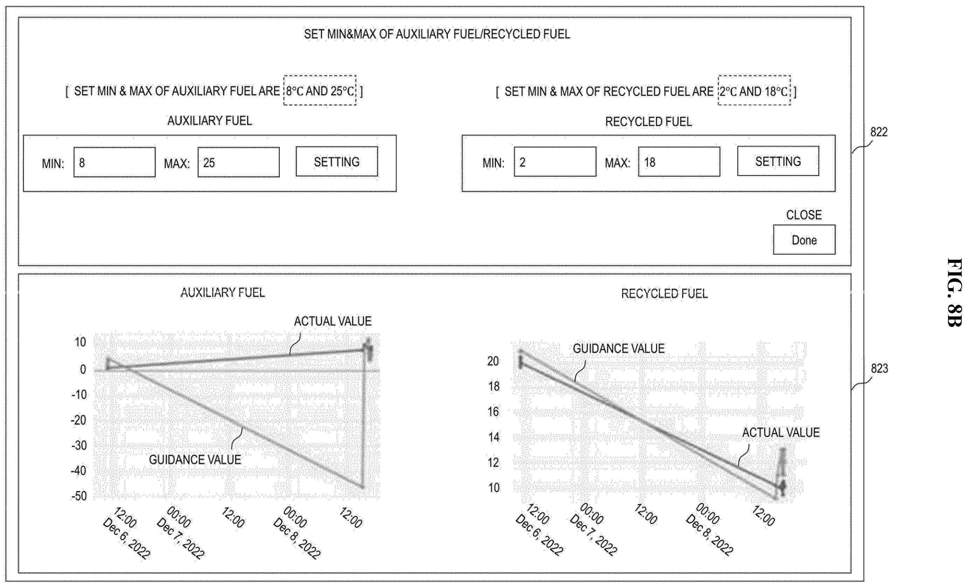

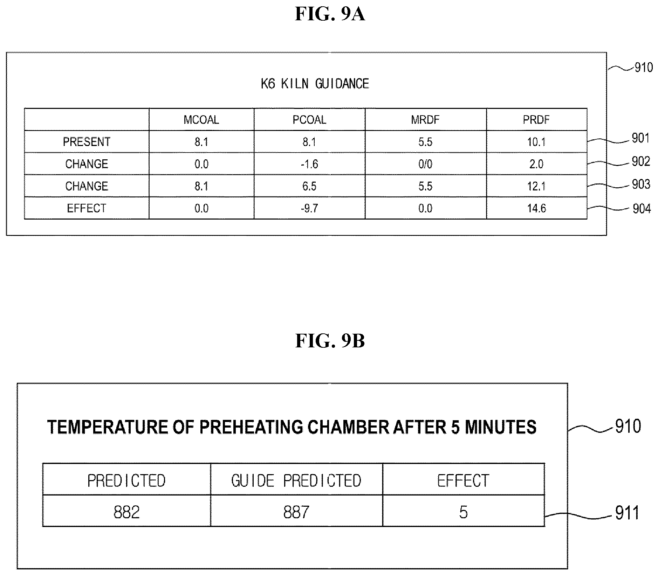

Next, according to one embodiment, in operation S640, the control method may provide a user interface (UI) including the obtained guidance information. A specific method of providing a UI will be described in detail with reference to FIGS. 8, 9A, and 9B.

FIG. 7 is a diagram illustrating a method of obtaining and providing guidance information according to one embodiment.

Referring to FIG. 7, according to one embodiment, in operation S710, the control method may first identify sub-fuel input information in which the input amount corresponding to each input fuel included in the fuel input information has changed. Here, the sub-fuel input information may be process information in which the ratio of the input fuel has been changed by a predetermined value, but is not limited thereto. According to one embodiment, the sub-fuel input information may be process information in which the process state (e.g., the pressure level within the cement manufacturing apparatus) has changed. For example, the processor 130 may identify the sub-fuel input information in which the input amount of the main fuel or auxiliary fuel is reduced by a predetermined amount.

Next, according to one embodiment, in operation S720, the control method may input the sub-fuel input information into the trained first neural network model to obtain second sub-predicted temperature information.

Next, according to one embodiment, in operation S730, the control method may obtain guidance information on the input fuel using the obtained second predicted temperature information and the second sub-predicted temperature information. Here, the guidance information refers to information on a recommended input ratio corresponding to each input fuel to increase the ratio of the input recycled fuel while the temperature of the first preheating chamber maintains the target temperature value. According to one embodiment, the processor 130 may obtain the guidance information on the input fuel through a data set containing the obtained second predicted temperature information and process information corresponding to the second predicted temperature information and a data set containing the second sub-predicted temperature information and process information corresponding to the second predicted temperature information, using a predetermined algorithm.

However, the present disclosure is not limited thereto. According to one embodiment, obviously, the processor 130 may obtain the guidance information on the input fuel based on information on a change value of the temperature of the first preheating chamber according to the change in the unit input amount corresponding to each of a plurality of types of input fuel including main fuel and auxiliary fuel.

Alternatively, according to one embodiment, the processor 130 may identify the guidance information on the input fuel based on at least one of temperature history information of the first preheating chamber and process environment information on the cement manufacturing apparatus 1, and provide the identified guidance information. According to one embodiment, the processor 130 may identify the guidance information on the input fuel based on at least one of the obtained second predicted temperature information, the second sub-predicted temperature information, the temperature history information of the first preheating chamber, and the process environment information on the cement manufacturing apparatus 1. Here, the process environment information may include at least one of the oxygen concentration, carbon monoxide concentration, carbon dioxide concentration, and nitrogen oxide concentration in the exhaust gas discharged from the inlet of the kiln within the cement manufacturing apparatus 1.

According to one embodiment, when the current temperature value of the first preheating chamber is identified based on the temperature history information of the first preheating chamber, the processor 130 may identify the guidance information based on a different value between the current temperature value of the first preheating chamber and the target temperature value. For example, when the difference value between the current temperature value of the first preheating chamber and the target temperature value is greater than and equal to a threshold value, the processor 130 may identify guidance information to ensure that the time for the temperature value of the first preheating chamber to reach the target temperature value is minimized while the temperature of the first preheating chamber maintains the target temperature value.

Alternatively, according to one embodiment, the processor 130 may identify the current temperature value of the first preheating chamber based on the temperature history information of the first preheating chamber and the temperature value of the first heating chamber at a first time point before a predetermined time from the present, and provide guidance information based on the identified temperature values.

For example, the processor 130 may identify the current temperature value of the first preheating chamber and the temperature value (or the past temperature value of the first preheating chamber) of the first preheating chamber one minute ago from the present, and compare the identified current temperature value of the first preheating chamber and the past temperature value of the first preheating chamber to obtain trend information on the temperature of the first preheating chamber. When the time for the temperature value of the first preheating chamber to reach a target temperature value is less than a threshold value based on the obtained trend information, the processor 130 may identify guidance information to ensure that the time for the temperature value of the first preheating chamber to reach the target temperature value is increased while the temperature of the first preheating chamber maintains the target temperature value. Alternatively, when the time for the temperature value of the first preheating chamber to reach a target temperature value is equal to or greater than the threshold value based on the obtained trend information, the processor 130 may identify guidance information to ensure that the time for the temperature value of the first preheating chamber to reach the target temperature value is reduced while the temperature of the first preheating chamber maintains the target temperature value.

Alternatively, according to one embodiment, the processor 130 may identify guidance information using the process environment information on the cement manufacturing apparatus 1. For example, when the concentration of carbon monoxide (CO) in the exhaust gas discharged from the inlet of the kiln is identified as exceeding a predetermined value, the processor 130 may update the guidance information to ensure that the input amount of recycled fuel is reduced compared to the previously identified input amount of the recycled fuel while the temperature of the first preheating chamber maintains the target temperature value. In other words, when the calcination state of the kiln is not optimal, such as when the concentration of carbon monoxide is equal to or greater than the predetermined value, the processor 130 may provide guidance information to the user to reduce the input amount of recycled fuel, thereby guiding the input amount of recycled fuel in consideration of the calcination state of the kiln.

However, when the magnitude of the current applied to a main motor included in the kiln is identified as being equal to or greater than a predetermined value based on the process environment information, the processor 130 may provide guidance information to maintain the previously identified input amount of recycled fuel. This is because the current value of the main motor of the kiln exceeding a predetermined value indicates that the calcination state of the kiln is optimal.

Alternatively, for example, the processor 130 may identify guidance information using the oxygen concentration in the exhaust gas included in the process environment information. Specifically, the processor 130 may identify trend information of the oxygen concentration in the exhaust gas based on the process environment information, and provide guidance information to reduce the input amount of recycled fuel when the oxygen concentration in the exhaust gas is identified as decreasing over time. In other words, when the oxygen concentration in the exhaust gas decreases, it indicates that the temperature in the preheating chamber increases. Therefore, the processor 130 may identify the amount of recycled fuel to be input based on the oxygen concentration in the exhaust gas.

However, the present disclosure is not limited thereto, and according to one embodiment, the processor 130 may identify the guidance information using a predetermined algorithm. For example, the processor 130 may provide a function for feedback control that causes the output to maintain a reference value based on an error between a control variable and a reference input. The processor 130 may identify guidance information on input fuel by using a target temperature value of the first preheating chamber, the current temperature value, and information for identifying the temperature value of the first preheating chamber (e.g., including information on control variables corresponding to different types of cement manufacturing apparatuses 1, including process information, such as information on the input amounts of main fuel, auxiliary fuel, and recycled fuel, the concentrations of oxygen, carbon dioxide, and nitrogen oxides in the exhaust gas at the kiln inlet, temperature information of the air fed into the pyro-rotor and kiln after cooling, the pressure of a fan within a cooler, the speed of a conveyor belt, and the speed information of the pyro-rotor and kiln, etc.).

Next, in operation S740, according to one embodiment, the control method may provide guide information including the obtained guidance information.

FIGS. 8A and 8B are diagrams illustrating a method of providing a UI according to one embodiment.

Referring to FIGS. 8A and 8B, according to one embodiment, the processor may provide a UI 800. According to one embodiment, the electronic device 100 may include a display (not shown), and the processor 130 may provide the UI 800 through the display (not shown).

In addition, according to one embodiment, the processor 130 may identify temperature history information of the first preheating chamber based on information stored in the memory 120, and based on this, provide the UI 800 including graph information corresponding to the temperature history of the first preheating chamber. In this case, according to one embodiment, the processor 130 may provide the UI 800 including graph information for comparing the obtained second predicted temperature information with the temperature history information of the first preheating chamber.

Meanwhile, according to one embodiment, the processor 130 may provide the obtained guidance information together with the graph information for comparing the second predicted temperature information with the temperature history information of the first preheating chamber.

In this case, according to one embodiment, the processor 130 may also provide effect information 904 corresponding to a change in the temperature of the preheating chamber predicted based on the guidance information. Here, the effect information 904 includes a contribution value to the temperature change corresponding to each of the plurality of types of fuel.

Meanwhile, according to one embodiment, the processor 130 may also provide a UI 810 for receiving a target temperature value of the first preheating chamber. Here, the guidance information refers to information on a recommended input ratio corresponding to each input fuel to increase the ratio of recycled fuel input while the temperature of the first preheating chamber maintains the target temperature value. The processor 130 may provide the UI 810 for receiving the target temperature value corresponding to the first preheating chamber from the user together with the UI 800 including the graph information for comparing the obtained second predicted temperature information with the temperature history information of the first preheating chamber. The processor 130 may receive information on the target temperature value from the user based on the provided UI 810 and perform an operation based on the received information.

Meanwhile, according to one embodiment, the processor 130 may provide a UI for providing history information corresponding to each of a plurality of types of input fuel and a UI 820 for receiving a user input corresponding to the maximum and minimum values for each input amount of main fuel or auxiliary fuel. According to one embodiment, the processor 130 may provide the above-described UI 820 through the display (not shown), and may provide the UI corresponding to FIG. 8B below when a user input corresponding to the UI 820 is received. This will be described in detail with reference to FIG. 8C.

Referring to FIG. 8B, according to one embodiment, the processor 130 may provide the UI for providing history information corresponding to each of the plurality of types of input fuel and the UI 820 for receiving the user input corresponding to the maximum and minimum values for each input amount of main fuel or auxiliary fuel. When the user input is received as the UI 820 is provided, the processor 130 may provide a UI 823 for providing history information corresponding to each of the plurality of types of input fuel and a UI 822 for receiving the user input corresponding to the maximum and minimum values for each input amount of main fuel or auxiliary fuel. In this case, when the user input corresponding to the maximum and minimum values for the input amount of main fuel or auxiliary fuel, respectively, is received through the UI 822 corresponding to the user input corresponding to the maximum and minimum values for each of the input among of the main fuel or auxiliary fuel, the processor 130 may input information corresponding to the received user input into a trained neural network model and thereby obtain guidance information reflecting the received user input.

Meanwhile, according to one embodiment, the process information may include process history information including temperature history information of the first preheating chamber and history information of input fuel. According to one embodiment, the processor 130 may identify history information on the input amount corresponding to each of the plurality of types of input fuel based on the information stored in the memory 120. Based on this, the processor 130 may provide a UI 823 including graph information corresponding to the input history of at least one of the main fuel or the auxiliary fuel.

Meanwhile, according to one embodiment, the UI 823 corresponding to the history information corresponding to each of the plurality of types of input fuel and the UI 822 corresponding to the user input corresponding to the maximum and minimum values for each input amount of main fuel or auxiliary fuel may be provided separately, but the present disclosure is not limited thereto. Obviously, the above-described UIs 822 and 823 may be provided together.

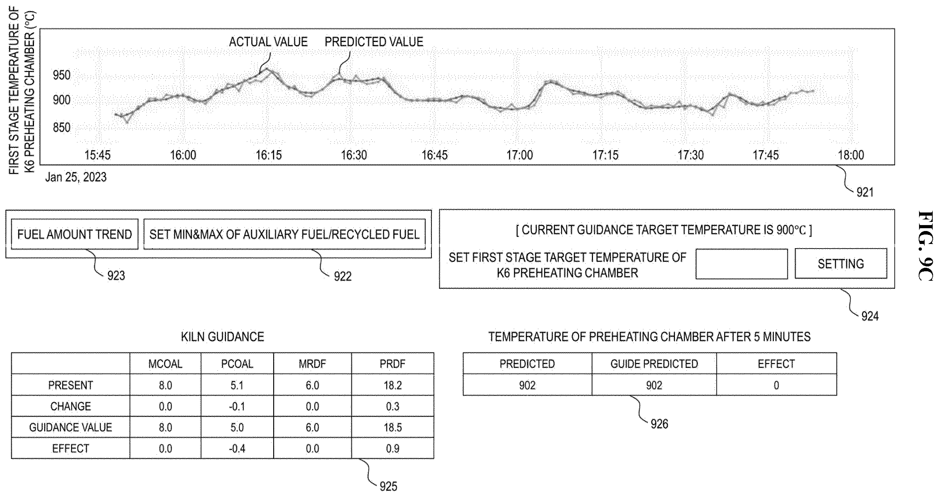

FIGS. 9A, 9B, and 9C are drawings illustrating a method of providing a UI according to one embodiment.

Referring to FIG. 9A, according to one embodiment, the processor 130 may provide a UI 900 including guidance information. According to one example, the UI 900 may include input amount information 901 corresponding to each of a plurality of types of input fuel currently being input. "MCOAL", "PCOAL", "MRDF", and "PRDF" shown in FIG. 9A may be examples of the plurality of types of input fuel, but are not limited thereto. Alternatively, according to one example, the UI 900 may include obtained guidance information 903. The guidance information 903 refers to information on a recommended input ratio corresponding to each input fuel to increase the ratio of the input recycled fuel while the temperature of the first preheating chamber is maintained in a predetermined range. Meanwhile, according to one example, the UI 900 may also provide change information 902 corresponding to a difference value between the input amount information 901 corresponding to each input fuel of the plurality of types of input fuel currently being input and the obtained guidance information 903.

Referring to FIG. 9B, according to one embodiment, the processor 130 may provide a UI 910 including information 911 on a temperature improvement value of the preheating chamber that will be improved when fuel is input based on the guidance information, and a temperature improvement value of the preheating chamber after a predetermined time (e.g., 5 minutes) corresponding to the currently input fuel, regardless of the guidance information, based on the obtained guidance information. In this case, the UI 910 containing the current temperature value of the preheating chamber and the temperature value of the preheating chamber predicted through a neural network model may be provided. Meanwhile, the temperature improvement value of the preheating chamber after a predetermined time (e.g., 5 minutes) corresponding to the currently input fuel refers to the value for the predicted temperature of the preheating chamber after a predetermined time based on the currently input fuel, regardless of the guidance information. The temperature improvement value of the preheating chamber that will be improved when fuel is input based on the guidance information refers to the value of the predicted temperature of the preheating chamber after a predetermined time in the case of fuel input based on the guidance information.

In addition, according to one embodiment, the processor 130 may provide information on a difference value between the temperature improvement value of the preheating chamber that will be improved when fuel is input based on the guidance information and the temperature improvement value of the preheating chamber after a predetermined time (e.g., 5 minutes) corresponding to the currently input fuel regardless of the guidance information, as "effect" information.

FIG. 9C illustrates an example of a user interface for providing a control method according to the present disclosure.

Portion 921 in FIG. 9C illustrates an example of a UI element for displaying temperature information of the preheating chamber. Temperature information may be provided in the form of a graph reflecting time information, such as portion 921, and may be provided in a form that makes it easy to identify comparison between actually measured information and predicted information.

Portion 922 in FIG. 9C illustrates an example of a UI element for providing the function of setting the fuel input amount. The user may set minimum and/or maximum values for the amount of input fuel through portion 922. At this time, the fuel may include a plurality of types, and the user may set the input amount of each of auxiliary fuel and/or recycled fuel through portion 922.

Portion 923 in FIG. 9C is an example of a UI element for providing fuel input amount information. The fuel input amount information may be provided in the form of a graph reflecting time information, and may be provided in a form that makes it easy to identify comparison between an actual value and a guidance value. At this time, the fuel may include a plurality of types, and the user may check the input amount of each auxiliary fuel and/or recycled fuel through portion 923.

Portion 924 in FIG. 9C is an example of a UI element for providing the function of setting the temperature of the preheating chamber. The user may set a preheating chamber target temperature through portion 924.

Portion 925 in FIG. 9C is an example of a UI element for providing guidance information. Portion 925 may include first information on the current fuel input amount, second information on a recommended fuel input amount to increase the ratio of recycled fuel input while the temperature of the preheating chamber is maintained in a predetermined range, and change information corresponding to a difference value between the first information and the second information.

At this time, the fuel may include a plurality of types, and "MCOAL," "PCOAL," "MRDF," and "PRDF" shown at 925 may be examples of the plurality of types of input fuel, but the present disclosure is not limited thereto. Furthermore, portion 925 may include information on the degree to which the plurality of types of fuel contribute to temperature change.

Portion 926 in FIG. 9C is an example of a UI element for providing predicted temperature information. Portion 926 may include information on the temperature predicted value of the preheating chamber after a predetermined time (5 minutes in an example of FIG. 9C) corresponding to the currently input fuel, information on the temperature predicted value of the preheating chamber after the predetermined time when the fuel is input based on the guidance information, and/or information on the amount of temperature change.

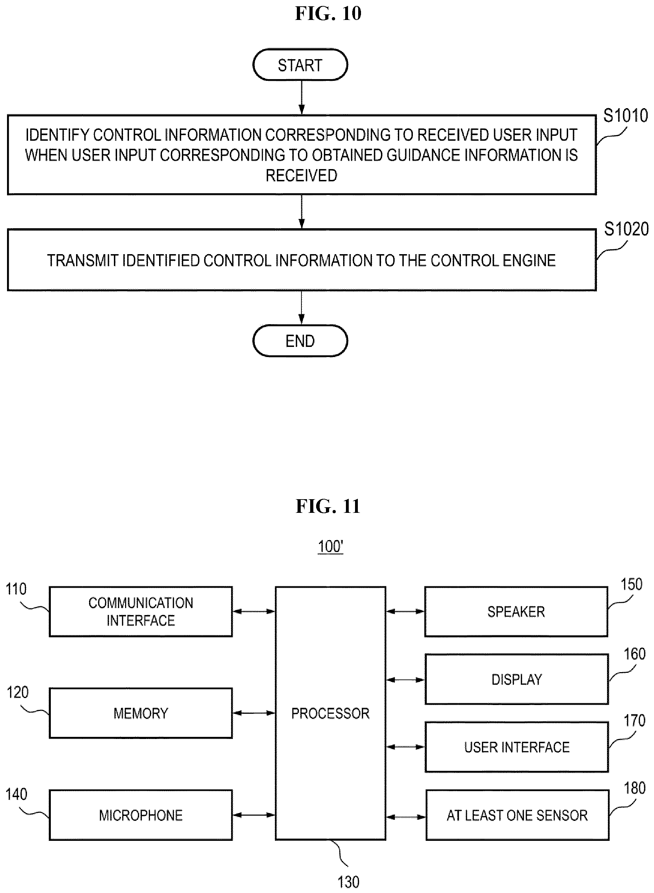

FIG. 10 is a diagram illustrating a method of identifying control information and transmitting the identified control information to a control engine according to one embodiment.

Referring to FIG. 10, according to one embodiment, when a user input corresponding to obtained guidance information is received, the control method may identify control information corresponding to the received user input in operation S1010. Here, the control information refers to a control signal for controlling the control engine, and the control engine refers to an engine for controlling the cement manufacturing apparatus 1. According to one embodiment, the electronic device 100 may further include a user interface (not shown), and the processor 130 may receive a user input corresponding to the obtained guidance information through the user interface (not shown). Next, the processor 130 may identify control information corresponding to the received user input based on the information stored in the memory 120.

Next, according to one embodiment, the control method may transmit the identified control information to the control engine in operation S1020. According to one embodiment, the processor 130 may transmit the identified control information to the control engine through the communication interface 110.

According to the above-described embodiment, the temperature of the first preheating chamber predicted in consideration of the calorific value of the recycled fuel may be identified, and based on the identified temperature, guidance information for an efficient process of the cement manufacturing apparatus 1 may be provided to the user. Accordingly, the implementation of environmentally friendly processes and carbon reduction processes can be more easily achieved.

FIG. 11 is a block diagram illustrating the detailed configuration of an electronic device according to one embodiment.

Referring to FIG. 11, an electronic device 100' may include a communication interface 110, a memory 120, one or more processors 130, a microphone 140, a speaker 150, a display 160, a user interface 170, and at least one sensor 180. Among the components shown in FIG. 11, detailed descriptions of components that overlap with those shown in FIG. 2 will be omitted.

The microphone 140 may refer to a module that acquires sound and converts the acquired sound into an electrical signal, and may be a condenser microphone, ribbon microphone, moving coil microphone, piezoelectric element microphone, carbon microphone, or a micro electro mechanical system (MEMS) microphone. In addition, the microphone 140 can be implemented in omni-directional, bi-directional, uni-directional, sub-cardioid, super-cardioid, and hyper-cardioid methods.

There may be various embodiments in which the electronic device 100' performs an operation corresponding to a user voice signal received through the microphone 140.

For example, the electronic device 100' may control the display 160 based on the user voice signal received through the microphone 140. For example, when a user voice signal for displaying content A is received, the electronic device 100' may control the display 160 to display content A.

For another example, the electronic device 100' may control an external display device connected to the electronic device 100' based on the user voice signal received through the microphone 140. Specifically, the electronic device 100' may provide a control signal for controlling the external display device to allow an operation corresponding to the user voice signal to be performed in the external display device, and transmit the provided control signal to the external display device. Here, the electronic device 100' may store a remote control application for controlling the external display device. In addition, the electronic device 100' may transmit the provided control signal to the external display device using at least one communication method of Bluetooth, Wi-Fi, and infrared. For example, when the user voice signal for displaying content A is received, the electronic device 100' may transmit a control signal for controlling content A to be displayed on the external display device, to the external display device. Here, the electronic device 100' may refer to various terminal devices that can install a remote control application, such as a smartphone or AI speaker.

As still another example, the electronic device 100' may use a remote control device to control an external display device connected to the electronic device 100' based on the user voice signal received through the microphone 140. Specifically, the electronic device 100' may transmit the control signal for controlling the external display device to the remote control device to allow the operation corresponding to the user voice signal to be performed on the external display device. The remote control device may transmit the control signal received from the electronic device 100' to the external display device. For example, when the user voice signal for displaying content A is received, the electronic device 100' may transmit the control signal for controlling content A to be displayed on an external display device to the remote control device, and the remote control device may transmit the received control signal to the external display device.

The speaker 150 may include a tweeter for reproducing high-pitched sounds, a midrange for reproducing mid-pitched sounds, a woofer for reproducing low-pitched sounds, a subwoofer for reproducing extremely low-pitched sounds, an enclosure for controlling resonance, and a crossover network that divides the frequency of an electrical signal input to the speaker by band.

The speaker 150 may output an acoustic signal to the outside of the electronic device 100'. The speaker 150 may output multimedia playback, recording playback, various notification sounds, voice messages, etc. The electronic device 100' may include an audio output device such as the speaker 150, or include an output device such as an audio output terminal. In particular, the speaker 150 may provide acquired information, information processed and produced based on the acquired information, response results to the user voice inputs, or operation results, etc., in the form of audio.

The display 160 may be implemented as a display including a self-emitting device or a display including a non-self-emitting device and a backlight. For example, the display 160 can be implemented as various types of displays, such as a liquid crystal display (LCD), an organic light emitting diode (OLED) display, light emitting diodes (LED), micro LEDs, mini LEDs, a plasma display panel (PDP), a quantum dot (QD) display, quantum dot light-emitting diodes (QLEDs), and the like. The display 160 may also include a driving circuit that may be implemented in the form of an a-si thin film transistor (TFT), a low temperature poly silicon (LTPS) TFT, or an organic TFT (OTFT), a back light unit, and the like. Meanwhile, the display 160 may be implemented as a touch screen combined with a touch sensor, a flexible display, a rollable display, a 3D display, or a display in which a plurality of display modules are physically connected. The processor 130 may control the display 160 to output the output image obtained according to the various embodiments described above. Here, the output image may be a high-resolution image of 4K or 8K or higher.

Meanwhile, according to another embodiment, the electronic device 100' may not include the display 160. The electronic device 100' may be connected to an external display device, and transmit images or content stored in the electronic device 100' to the external display device. Specifically, the electronic device 100' may transmit images or content to the external display device along with a control signal for controlling the images or content to be displayed on the external display device.

Here, the external display device may be connected to the electronic device 100' through a communication interface 110 or an input/output interface (not shown). For example, the electronic device 100' may not include a display, such as in a set top box (STB). In addition, the electronic device 100' may include only a small display capable of displaying only simple information such as text information. Here, the electronic device 100' may transmit the images or content to the external display device wired or wirelessly through the communication interface 110, or may transmit the images or content to the external display device through an input/output interface (not shown).

The user interface 170 is a component that allows the electronic device 100' to perform interaction with a user. For example, the user interface 170 may include at least one of a touch sensor, a motion sensor, a button, a jog dial, a switch, a microphone, and a speaker, but is not limited thereto.

At least one sensor 180 (hereinafter referred to as sensor) may include various types of a plurality of sensors. The sensor 180 may measure a physical quantity or detect the operating state of the electronic device 100', and convert the measured or detected information into an electrical signal. The sensor 180 may include a camera, and the camera may include a lens that focuses visible light or other optical signals reflected by an object and received onto an image sensor, and an image sensor that can detect visible light or other optical signals. Here, the image sensor may include a 2D pixel array divided into a plurality of pixels.

According to the above-described example, the electronic device 100' can predict the calorific value of the recycled fuel using the trained neural network model, and can predict the temperature of the first preheating chamber based on the predicted calorific value. Accordingly, the electronic device 100' can provide guidance information to the user based on the accurately predicted temperature of the preheating chamber, thereby enabling an eco-friendly and efficient process.

Meanwhile, the methods according to various embodiments of the present disclosure described above may be implemented in the form of applications that can be installed on existing electronic devices. Alternatively, the methods according to various embodiments of the present disclosure described above may be performed using a deep learning-based trained neural network (or deep trained neural network), that is, learning network model. In addition, the methods according to various embodiments of the present disclosure described above may be implemented only by software upgrade for an existing electronic device, or hardware upgrade. In addition, the various embodiments of the present disclosure described above may also be performed through an embedded server provided in an electronic device or an external server of the electronic device.

Meanwhile, according to one embodiment of the present disclosure, the various embodiments described above may be implemented as software including instructions stored in a storage medium that is readable by a machine (e.g., a computer). The device is a device capable of calling the stored instructions from the storage medium and operating according to the called instructions, and include a display device (e.g., display device A) according to the disclosed embodiments. When an instruction is executed by a processor, the processor may perform a function corresponding to the instruction directly or using other components under the control of the processor. The instruction may contain codes provided or executed by a compiler or interpreter. The machine-readable storage medium may be provided in the form of a non-transitory storage medium. Here, the term 'non-transitory' only means that a storage medium is a device that is tangible and does not include a signal, and the term does not distinguish between a case where data is semi-permanently stored and a case where it is temporarily stored.

In addition, according to one embodiment, the methods according to various embodiments described above may be provided by being included in a computer program product. The computer program products may be traded between sellers and buyers as products. The computer program products may be distributed in the form of machine-readable storage medium (e.g., compact disc read only memory (CD-ROM)) or distributed online through an application store (e.g. Play Store™). In the case of online distribution, at least some of the computer program products may be at least temporarily stored or temporarily provided on a storage medium such as a manufacturer's server, a server in an application store, or a memory in a relay server.

In addition, each component (e.g., module or program) according to the various embodiments described above may be composed of a single or multiple entities, and some of the sub-components described above may be omitted, or other sub-components may be included in various embodiments. Alternatively or additionally, some components (e.g., modules or programs) may be integrated into a single entity and perform the same or similar functions performed by each corresponding component prior to integration. According to various embodiments, operations performed by a module, program, or other component may be executed sequentially, in parallel, iteratively, or heuristically, or at least some operations may be executed in a different order, omitted, or other operations may be added.

As described above, according to the present disclosure, using an artificial intelligence model trained based on data on the manufacturing process, the calorific value of the recycled fuel that can reduce carbon emissions can be predicted, and the temperature of the preheating chamber can be accurately predicted using the predicted calorific value of recycled fuel. In addition, according to the present disclosure, it is possible to provide guidance information for maintaining an appropriate processing temperature to a user based on an accurately predicted temperature.

In the above, exemplary embodiments of the present disclosure have been shown and described, but the present disclosure is not limited to the specific embodiments described above, and may be used in the technical field pertaining to the disclosure without departing from the scope of the claims. Of course, various modifications can be made by those skilled in the art, and these modifications should not be understood individually from the technical ideas or perspectives of the present disclosure.

**Claims**

**1.** An electronic device (100) for implementing a temperature prediction and control system, the electronic device (100) comprising:

a communication interface (110);
a memory (120) in which a trained first neural network model and a trained second neural network model are stored; and
one or more processors (130) configured to:

perform preprocessing on, when process information (50) including fuel input information (40) of a cement manufacturing apparatus (1) is received through the communication interface (110), the received process information (50);
input a data set containing i) the preprocessed process information (50), ii) first predicted temperature information output from the trained first neural network model and iii) measured temperature of a first preheating chamber in the cement manufacturing apparatus (1) into the trained second neural network model to obtain error information between the first predicted temperature information and the measured temperature of the first preheating chamber, wherein the measured temperature of the first preheating chamber is included in preprocessed process information (50);
identify predicted calorific information of a first recycled fuel based on the obtained error information and the fuel input information (40), wherein information on the first recycled fuel is included in the fuel input information (40);
update the fuel input information (40) based on the identified predicted calorific information;
input the updated fuel input information (40) into the trained first neural network model to obtain second predicted temperature information of the first preheating chamber; and
provide a user interface, UI, including guidance information for maintaining a target temperature of the first preheating chamber, wherein the guidance information corresponds to the obtained second predicted temperature information.

2. The electronic device (100) of claim 1, wherein, when information on an input amount corresponding to each of different types of input fuels including the first recycled fuel and the fuel input information (40) including calorific information corresponding to each of the input fuels are input, the trained first neural network model is trained to output predicted temperature information of the first preheating chamber.

3. The electronic device (100) of claim 1,

wherein the fuel input information (40) includes information on an input amount corresponding to each of different types of input fuels including at least one of the first recycled fuel, main fuel, and auxiliary fuel, and calorific information corresponding to each input amount, and
the one or more processors (130) are further configured to:

receive a target temperature value of the first preheating chamber in the cement manufacturing apparatus (1) through the UI;
identify the input amount corresponding to each of the different types of input fuels to ensure that the temperature value of the first preheating chamber reaches the target temperature value based on the obtained second predicted temperature information and the received target temperature value;
obtain guidance information corresponding to the identified input amount; and
provide a UI including the obtained guidance information.

4. The electronic device (100) of claim 3, wherein the one or more processors (130) are further configured to:

identify sub-fuel input information in which the input amount corresponding to each input fuel included in the fuel input information (40) has changed;
input the sub-fuel input information into the trained first neural network model to obtain second-sub predicted temperature information:

obtain guidance information on the input fuel using the obtained second predicted temperature information and the second sub-predicted temperature information; and
provide guide information including the obtained guidance information.

5. The electronic device (100) of claim 3, wherein the preprocessed process information (50) includes temperature history information of the first preheating chamber and history information of the input fuel, and
the one or more processors (130) provide a UI including the received temperature history information of the first preheating chamber, the history information of the input fuel, and the obtained second predicted temperature information.

**6.** The electronic device (100) of claim 1, wherein the one or more processors (130) identify predicted calorific information of the first recycled fuel based on the obtained error information and information on an input amount of the first recycled fuel included in the fuel input information (40).

**7.** The electronic device (100) of claim 1,
wherein the one or more processors (130) are further configured to:

identify control information, when a user input corresponding to the guidance information is received through the UI, corresponding to the received user input; and
transmit the identified control information to a control engine through the communication interface (110).

**8.** A control method of an electronic device (100) for implementing a temperature prediction and control system, the control method comprising:

performing preprocessing on, when process information (50) including fuel input information (40) of a cement manufacturing apparatus (1) is received, the received process information (50);
inputting a data set containing i) the preprocessed process information (50), ii) first predicted temperature information output from a trained first neural network model and iii) measured temperature of a first preheating chamber in the cement manufacturing apparatus (1) into a trained second neural network model to obtain error information between the first predicted temperature information and the measured temperature of the first preheating chamber, wherein the measured temperature of the first preheating chamber is included in preprocessed process information (50);
identifying predicted calorific information of a first recycled fuel based on the obtained error information and the fuel input information (40), wherein information on the first recycled fuel is included in the fuel input information (40);
update the fuel input information (40) based on the identified predicted calorific information;
inputting the updated fuel input information (40) into the trained first neural network model to obtain second predicted temperature information of the first preheating chamber; and
providing a user interface, UI, including guidance information for maintaining a target temperature of the first preheating chamber, wherein the guidance information corresponds to the obtained second predicted temperature information.

**9.** The control method of claim 8, wherein, when information on an input amount corresponding to each of different types of input fuels including the first recycled fuel and the fuel input information (40) including calorific information corresponding to each of the input fuels are input, the trained first neural network model is trained to output predicted temperature information of the first preheating chamber.

**10.** The control method of claim 8, wherein the fuel input information (40) includes information on an input amount corresponding to each of different types of input fuels including at least one of the first recycled fuel, main fuel, and auxiliary fuel, and calorific information corresponding to each input amount, and the providing of the guidance information includes:

receiving a target temperature value of the first preheating chamber in the cement manufacturing apparatus (1) through the UI;
identifying the input amount corresponding to each of the different types of input fuels to ensure that the temperature value of the first preheating chamber reaches the target temperature value based on the obtained second predicted temperature information and the received target temperature value; and
obtaining guidance information corresponding to the identified input amount, and
the control method further comprising providing a UI including the obtained guidance information.

**11.** The control method of claim 10, wherein the obtaining of the guidance information further includes:

identifying sub-fuel input information in which the input amount corresponding to each input fuel included in the fuel input information (40) has changed;
inputting the sub-fuel input information into the trained first neural network model to obtain second-sub predicted temperature information;
obtaining guidance information on the input fuel using the obtained second predicted temperature information and the second sub-predicted temperature information; and
providing guide information including the obtained guidance information.

**12.** The control method of claim 10, wherein the preprocessed process information (50) includes temperature history information of the first preheating chamber and history information of the input fuel, and the providing of the UI includes providing a UI including the received temperature history information of the first preheating chamber, the history information of the input fuel, and the obtained second predicted temperature information.

13. The control method of claim 8, wherein the identifying of the calorific information includes identifying predicted calorific information of the first recycled fuel based on the obtained error information and information on an input amount of the first recycled fuel included in the fuel input information (40).

14. The control method of claim 8, further comprising:

identifying control information, when a user input corresponding to the guidance information is received through the UI, corresponding to the received user input; and
transmitting the identified control information to a control engine.

15. A non-transitory computer-readable recording medium that stores, when executed by a processor (130) of an electronic device (100) for implementing a temperature prediction and control system, computer instructions that cause the electronic device (100) to perform operations of:

performing preprocessing on, when process information (50) including fuel input information (40) of a cement manufacturing apparatus (1) is received, the received process information (50);
inputting a data set containing i) the preprocessed process information (50), ii) first predicted temperature information output from a trained first neural network model and iii) measured temperature of a first preheating chamber in the cement manufacturing apparatus (1) into a trained second neural network model to obtain error information between the first predicted temperature information and the measured temperature of the first preheating chamber, wherein the measured temperature of the first preheating chamber is included in preprocessed process information (50);
identifying predicted calorific information of a first recycled fuel based on the obtained error information and the fuel input information (40), wherein information on the first recycled fuel is included in the fuel input information (40);
update the fuel input information (40) based on the identified predicted calorific information;
inputting the updated fuel input information (40) into the trained first neural network model to obtain second predicted temperature information of the first preheating chamber; and
providing a user interface, UI, including guidance information for maintaining a target temperature of the first preheating chamber, wherein the guidance information corresponds to the obtained second predicted temperature information.

**Patentansprüche**

1. Elektronische Vorrichtung (100) zur Implementierung eines Temperaturvorhersage- und Steuerungssystems, wobei die elektronische Vorrichtung (100) Folgendes umfasst:

eine Kommunikationsschnittstelle (110);
einen Speicher (120), in dem ein trainiertes erstes neuronales Netzwerkmodell und ein trainiertes zweites neuronales Netzwerkmodell gespeichert sind; und
einen oder mehrere Prozessoren (130), die zu Folgendem konfiguriert sind:

Ausführen einer Vorverarbeitung der empfangenen Prozessinformationen (50), wenn Prozessinformationen (50), die Kraftstoffeingabeinformationen (40) einer Zementherstellungsanlage (1) enthalten, über die Kommunikationsschnittstelle (110) empfangen werden;
Eingeben eines Datensatzes, der i) die vorverarbeiteten Prozessinformationen (50), ii) die von dem trainierten ersten neuronalen Netzwerkmodell ausgegebenen ersten vorhergesagten Temperaturinformationen und iii) die gemessene Temperatur einer ersten Vorwärmkammer in der Zementherstellungsanlage (1) enthält, in das trainierte zweite neuronale Netzwerkmodell, um Fehlerinformationen zwischen den ersten vorhergesagten Temperaturinformationen und der gemessenen Temperatur der ersten Vorwärmkammer zu erhalten, wobei die gemessene Temperatur der ersten Vorwärmkammer in den vorverarbeiteten Prozessinformationen (50) enthalten ist;
Identifizieren vorhergesagter Heizwertinformationen eines ersten recycelten Kraftstoffs basierend auf den erhaltenen Fehlerinformationen und den Kraftstoffeingabeinformationen (40), wobei die Informationen über den ersten recycelten Kraftstoff in den Kraftstoffeingabeinformationen (40) enthalten sind;
Aktualisieren der Kraftstoffeingabeinformationen (40) basierend auf den identifizierten vorhergesagten Heizwertinformationen;
Eingeben der aktualisierten Kraftstoffeingabeinformationen (40) in das trainierte erste neuronale Netzwerkmodell, um die zweiten vorhergesagten Temperaturinformationen der ersten Vorwärmkammer zu erhalten; und

Bereitstellen einer Benutzeroberfläche, UI, die Leitlinieninformationen zur Aufrechterhaltung einer Zieltemperatur der ersten Vorwärmkammer enthält, wobei die Leitlinieninformationen den erhaltenen zweiten vorhergesagten Temperaturinformationen entsprechen.

2. Elektronische Vorrichtung (100) nach Anspruch 1, wobei, wenn Informationen über eine Eingabemenge, die jeder der verschiedenen Arten von eingegebenen Kraftstoffen entspricht, die den ersten recycelten Kraftstoff enthalten, und die Kraftstoffeingabeinformationen (40) eingegeben werden, die Heizwertinformationen enthalten, die jedem der eingegebenen Kraftstoffe entsprechen, das trainierte erste neuronale Netzwerkmodell so trainiert wird, dass es vorhergesagte Temperaturinformationen der ersten Vorwärmkammer ausgibt.

3. Elektronische Vorrichtung (100) nach Anspruch 1,

wobei die Kraftstoffeingabeinformationen (40) Informationen über eine Eingabemenge, die jeder der verschiedenen Arten von eingegebenen Kraftstoffen entspricht, die den ersten recycelten Kraftstoff, den Hauptkraftstoff und/oder Hilfskraftstoff enthalten, und Heizwertinformationen enthalten, die jeder Eingabemenge entsprechen, und
der eine oder die mehreren Prozessoren (130) ferner zu Folgendem konfiguriert sind:

Empfangen eines Zieltemperaturwerts der ersten Vorwärmkammer in der Zementherstellungsanlage (1) über die UI;
Identifizieren der Eingabemenge, die jeder der verschiedenen Arten von eingegebenen Kraftstoffen entspricht, um sicherzustellen, dass der Temperaturwert der ersten Vorwärmkammer den Zieltemperaturwert erreicht, basierend auf den erhaltenen zweiten vorhergesagten Temperaturinformationen und dem empfangenen Zieltemperaturwert;
Erhalten von Leitlinieninformationen, die der identifizierten Eingabemenge entsprechen; und
Bereitstellen einer UI einschließlich der erhaltenen Leitlinieninformationen.

4. Elektronische Vorrichtung (100) nach Anspruch 3, wobei der eine oder die mehreren Prozessoren (130) ferner zu Folgendem konfiguriert sind:

Identifizieren von Teil-Kraftstoffeingabeinformationen, bei denen sich die Eingabemenge geändert hat, die jedem eingegebenen Kraftstoff entspricht, der in den Kraftstoffeingabeinformationen (40) enthalten ist;
Eingeben der Teil-Kraftstoffeingabeinformationen in das trainierte erste neuronale Netzwerkmodell, um zweite vorhergesagte Teil-Temperaturinformationen zu erhalten;
Erhalten von Leitlinieninformationen über den eingegebenen Kraftstoff unter Verwendung der erhaltenen zweiten vorhergesagten Temperaturinformationen und der zweiten vorhergesagten Teil-Temperaturinformationen; und
Bereitstellen von Leitlinieninformationen einschließlich der erhaltenen Leitlinieninformationen.

5. Elektronische Vorrichtung (100) nach Anspruch 3, wobei die vorverarbeiteten Prozessinformationen (50) Temperaturverlaufsinformationen der ersten Vorwärmkammer und Verlaufsinformationen des eingegebenen Kraftstoffs enthalten, und
der eine oder die mehreren Prozessoren (130) eine UI bereitstellen, die die empfangenen Temperaturverlaufsinformationen der ersten Vorwärmkammer, die Verlaufsinformationen des eingegebenen Kraftstoffs und die erhaltenen zweiten vorhergesagten Temperaturinformationen enthält.

6. Elektronische Vorrichtung (100) nach Anspruch 1, wobei der eine oder die mehreren Prozessoren (130) vorhergesagte Heizwertinformationen des ersten recycelten Kraftstoffs basierend auf den erhaltenen Fehlerinformationen und Informationen über eine Eingabemenge des ersten recycelten Kraftstoffs identifizieren, die in den Kraftstoffeingabeinformationen (40) enthalten ist.

7. Elektronische Vorrichtung (100) nach Anspruch 1, wobei der eine oder die mehreren Prozessoren (130) ferner zu Folgendem konfiguriert sind:

Identifizieren von Steuerungsinformationen, wenn eine Benutzereingabe, die den Leitlinieninformationen entspricht, über die UI empfangen wird, die der empfangenen Benutzereingabe entspricht; und
Übertragen der identifizierten Steuerungsinformationen über die Kommunikationsschnittstelle (110) an einen Steuerungsmotor.

8. Steuerungsverfahren einer elektronischen Vorrichtung (100) zur Implementierung eines Temperaturvorhersage- und Steuerungssystems, wobei das Steuerungsverfahren Folgendes umfasst:

Ausführen einer Vorverarbeitung der empfangenen Prozessinformationen (50), wenn Prozessinformationen (50) empfangen werden, die Kraftstoffeingabeinformationen (40) einer Zementherstellungsanlage (1) enthalten;
Eingeben eines Datensatzes, der i) die vorverarbeiteten Prozessinformationen (50), ii) die von einem trainierten ersten neuronalen Netzwerkmodell ausgegebenen ersten vorhergesagten Temperaturinformationen und iii) die gemessene Temperatur einer ersten Vorwärmkammer in der Zementherstellungsanlage (1) enthält, in ein trainiertes zweites neuronales Netzwerkmodell, um Fehlerinformationen zwischen den ersten vorhergesagten Temperaturinformationen und der gemessenen Temperatur der ersten Vorwärmkammer zu erhalten, wobei die gemessene Temperatur der ersten Vorwärmkammer in den vorverarbeiteten Prozessinformationen (50) enthalten ist;
Identifizieren vorhergesagter Heizwertinformationen eines ersten recycelten Kraftstoffs basierend auf den erhaltenen Fehlerinformationen und den Kraftstoffeingabeinformationen (40), wobei die Informationen über den ersten recycelten Kraftstoff in den Kraftstoffeingabeinformationen (40) enthalten sind;
Aktualisieren der Kraftstoffeingabeinformationen (40) basierend auf den identifizierten vorhergesagten Heizwertinformationen;
Eingeben der aktualisierten Kraftstoffeingabeinformationen (40) in das trainierte erste neuronale Netzwerkmodell, um zweite vorhergesagte Temperaturinformationen der ersten Vorwärmkammer zu erhalten; und
Bereitstellen einer Benutzeroberfläche, UI, die Leitlinieninformationen zur Aufrechterhaltung einer Zieltemperatur der ersten Vorwärmkammer enthält, wobei die Leitlinieninformationen den erhaltenen zweiten vorhergesagten Temperaturinformationen entsprechen.

9. Steuerungsverfahren nach Anspruch 8, wobei, wenn Informationen über eine Eingabemenge, die jeder der verschiedenen Arten von eingegebenen Kraftstoffen entspricht, die den ersten recycelten Kraftstoff enthalten, und die Kraftstoffeingabeinformationen (40) eingegeben werden, die die Heizwertinformationen enthalten, die jedem der eingegebenen Kraftstoffe entsprechen, das trainierte erste neuronale Netzwerkmodell so trainiert wird, dass es vorhergesagte Temperaturinformationen der ersten Vorwärmkammer ausgibt.

10. Steuerungsverfahren nach Anspruch 8, wobei die Kraftstoffeingabeinformationen (40) Informationen über eine Eingabemenge, die jeder der verschiedenen Arten von eingegebenen Kraftstoffen entspricht, die den ersten recycelten Kraftstoff, den Hauptkraftstoff und/oder den Hilfskraftstoff enthalten, und Heizwertinformationen enthalten, die jeder Eingabemenge entsprechen, und

das Bereitstellen der Leitlinieninformationen Folgendes umfasst:

Empfangen eines Zieltemperaturwerts der ersten Vorwärmkammer in der Zementherstellungsanlage (1) über die UI;
Identifizieren der Eingabemenge, die jeder der verschiedenen Arten von eingegebenen Kraftstoffen entspricht, um sicherzustellen, dass der Temperaturwert der ersten Vorwärmkammer den Zieltemperaturwert erreicht, basierend auf den erhaltenen zweiten vorhergesagten Temperaturinformationen und dem empfangenen Zieltemperaturwert; und
Erhalten von Leitlinieninformationen, die der identifizierten Eingabemenge entsprechen, und wobei das Steuerungsverfahren ferner das Bereitstellen einer UI umfasst, die die erhaltenen Leitlinieninformationen enthält.

11. Steuerungsverfahren nach Anspruch 10, wobei das Erhalten der Leitlinieninformationen ferner Folgendes enthält:

Identifizieren von Teil-Kraftstoffeingabeinformationen, bei denen sich die Eingabemenge geändert hat, die jedem eingegebenen Kraftstoff entspricht, der in den Kraftstoffeingabeinformationen (40) enthalten ist;
Eingeben der Teil-Kraftstoffeingabeinformationen in das trainierte erste neuronale Netzwerkmodell, um zweite vorhergesagte Teil-Temperaturinformationen zu erhalten;
Erhalten von Leitlinieninformationen über den eingegebenen Kraftstoff unter Verwendung der erhaltenen zweiten vorhergesagten Temperaturinformationen und der zweiten vorhergesagten Teil-Temperaturinformationen; und
Bereitstellen von Leitlinieninformationen einschließlich der erhaltenen Leitlinieninformationen.

12. Steuerungsverfahren nach Anspruch 10, wobei die vorverarbeiteten Prozessinformationen (50) Temperaturverlaufsinformationen der ersten Vorwärmkammer und Verlaufsinformationen des eingegebenen Kraftstoffs enthalten, und
das Bereitstellen der UI das Bereitstellen einer UI enthält, die die empfangenen Temperaturverlaufsinformationen der ersten Vorwärmkammer, die Verlaufsinformationen des eingegebenen Kraftstoffs und die erhaltenen zweiten vorhergesagten Temperaturinformationen enthält.

13. Steuerungsverfahren nach Anspruch 8, wobei das Identifizieren der Heizwertinformationen das Identifizieren vorhergesagter Heizwertinformationen des ersten recycelten Kraftstoffs basierend auf den erhaltenen Fehlerinformationen und Informationen über eine Eingabemenge des ersten recycelten Kraftstoffs enthält, die in den Kraftstoffeingabeinformationen (40) enthalten ist.

14. Steuerungsverfahren nach Anspruch 8, das ferner Folgendes umfasst:

    Identifizieren von Steuerungsinformationen, wenn eine Benutzereingabe, die den Leitlinieninformationen entspricht, über die UI empfangen wird, die der empfangenen Benutzereingabe entspricht; und
    Übertragen der identifizierten Steuerungsinformationen an einen Steuerungsmotor.

15. Nicht-transitorisches computerlesbares Aufzeichnungsmedium, das, wenn es von einem Prozessor (130) einer elektronischen Vorrichtung (100) zur Implementierung eines Temperaturvorhersage- und Steuerungssystems ausgeführt wird, Computeranweisungen speichert, die die elektronische Vorrichtung (100) veranlassen, die folgenden Vorgänge auszuführen:

    Ausführen einer Vorverarbeitung der empfangenen Prozessinformationen (50), wenn Prozessinformationen (50) empfangen werden, die die Kraftstoffeingabeinformationen (40) einer Zementherstellungsanlage (1) enthalten;
    Eingeben eines Datensatzes, der i) die vorverarbeiteten Prozessinformationen (50), ii) die von einem trainierten ersten neuronalen Netzwerkmodell ausgegebenen ersten vorhergesagten Temperaturinformationen und iii) die gemessene Temperatur einer ersten Vorwärmkammer in der Zementherstellungsanlage (1) enthält, in ein trainiertes zweites neuronales Netzwerkmodell, um Fehlerinformationen zwischen den ersten vorhergesagten Temperaturinformationen und der gemessenen Temperatur der ersten Vorwärmkammer zu erhalten, wobei die gemessene Temperatur der ersten Vorwärmkammer in den vorverarbeiteten Prozessinformationen (50) enthalten ist;
    Identifizieren vorhergesagter Heizwertinformationen eines ersten recycelten Kraftstoffs basierend auf den erhaltenen Fehlerinformationen und den Kraftstoffeingabeinformationen (40), wobei Informationen über den ersten recycelten Kraftstoff in den Kraftstoffeingabeinformationen (40) enthalten sind;
    Aktualisieren der Kraftstoffeingabeinformationen (40) basierend auf den identifizierten vorhergesagten Heizwertinformationen;
    Eingeben der aktualisierten Kraftstoffeingabeinformationen (40) in das trainierte erste neuronale Netzwerkmodell, um zweite vorhergesagte Temperaturinformationen der ersten Vorwärmkammer zu erhalten; und
Bereitstellen einer Benutzeroberfläche, UI, die Leitlinieninformationen zur Aufrechterhaltung einer Zieltemperatur der ersten Vorwärmkammer enthält, wobei die Leitlinieninformationen den erhaltenen zweiten vorhergesagten Temperaturinformationen entsprechen.

**Revendications**

1. Dispositif électronique (100) destiné à mettre en œuvre un système de prévision et de régulation de la température, le dispositif électronique (100) comprenant :

    une interface de communication (110) ;
    une mémoire (120) dans laquelle sont stockés un premier modèle de réseau neuronal entraîné et un deuxième modèle de réseau neuronal entraîné ; et
    un ou plusieurs processeurs (130) configurés pour :

        effectuer un prétraitement, lorsque des informations de processus (50) comprenant des informations sur l'apport de combustible (40) d'un appareil de fabrication de ciment (1) sont reçues via l'interface de communication (110), des informations de processus reçues (50) ;
        introduire, dans le deuxième modèle de réseau neuronal entraîné, un ensemble de données contenant i) les informations de processus prétraitées (50), ii) des premières informations de température prédite fournies par le premier modèle de réseau neuronal entraîné et iii) la température mesurée d'une première chambre de préchauffage dans l'appareil de fabrication de ciment (1) afin d'obtenir des informations d'erreur entre les premières informations de température prédite et la température mesurée de la première chambre de préchauffage, où la température mesurée de la première chambre de préchauffage est incluse dans les informations de processus prétraitées (50) ;
        identifier des informations calorifiques prédites d'un premier combustible recyclé sur la base des informations d'erreur obtenues et des informations sur l'apport de combustible (40), où les informations sur le premier combustible recyclé sont incluses dans les informations sur l'apport de combustible (40) ;
        mettre à jour les informations sur l'apport de combustible (40) sur la base des informations calorifiques prédites identifiées ;
introduire les informations sur l'apport de combustible actualisées (40) dans le premier modèle de réseau neuronal entraîné afin d'obtenir des deuxièmes informations de température prédite de la première chambre de préchauffage ; et
fournir une interface utilisateur, UI, comprenant des informations de guidage pour maintenir une température cible de la première chambre de préchauffage, où les informations de guidage correspondent aux deuxièmes informations de température prédite obtenues.

2. Dispositif électronique (100) selon la revendication 1, où, lorsque des informations sur une quantité d'apport correspondant à chacun des différents types de combustibles apportés, y compris le premier combustible recyclé, et les informations sur l'apport de combustible (40) comprenant des informations calorifiques correspondant à chacun des combustibles apportés sont introduites, le premier modèle de réseau neuronal entraîné est entraîné pour fournir des informations de température prédite de la première chambre de préchauffage.

3. Dispositif électronique (100) selon la revendication 1,

où les informations sur l'apport de combustible (40) comprennent des informations sur une quantité d'apport correspondant à chacun des différents types de combustibles apportés, comprenant au moins l'un parmi le premier combustible recyclé, le combustible principal et le combustible auxiliaire, ainsi que des informations calorifiques correspondant à chaque quantité d'apport, et
l'un ou plusieurs processeurs (130) sont en outre configurés pour :

recevoir une valeur de température cible de la première chambre de préchauffage dans l'appareil de fabrication de ciment (1) via l'UI ;
identifier la quantité d'apport correspondant à chacun des différents types de combustibles apportés afin de garantir que la valeur de température de la première chambre de préchauffage atteigne la valeur de température cible sur la base des deuxièmes informations de température prédite obtenues et de la valeur de température cible reçue ;
obtenir des informations de guidage correspondant à la quantité d'apport identifiée ; et

fournir une UI comprenant les informations de guidage obtenues.

4. Dispositif électronique (100) selon la revendication 3, où l'un ou plusieurs processeurs (130) sont en outre configurés pour :

identifier des sous-informations sur l'apport de combustible dans lesquelles la quantité d'apport correspondant à chaque apport de combustible incluse dans les informations sur l'apport de combustible (40) a changé ;
introduire les sous-informations sur l'apport de combustible dans le premier modèle de réseau neuronal entraîné afin d'obtenir des deuxièmes sous-informations de température prédite :

obtenir des informations de guidage sur le combustible apporté en utilisant les deuxièmes sous-informations de température prédite obtenues et les deuxièmes sous-informations de température prédite ; et
fournir des informations de guidage comprenant les informations de guidage obtenues.

5. Dispositif électronique (100) selon la revendication 3, où les informations de processus prétraitées (50) comprennent des informations d'historique de température de la première chambre de préchauffage et des informations d'historique du combustible apporté, et
l'un ou plusieurs processeurs (130) fournissent une UI comprenant les informations d'historique de température reçues de la première chambre de préchauffage, les informations d'historique du combustible apporté et les deuxièmes informations de température prédite obtenues.

6. Dispositif électronique (100) selon la revendication 1, où l'un ou plusieurs processeurs (130) identifient des informations calorifiques prévues du premier combustible recyclé sur la base des informations d'erreur obtenues et des informations relatives à la quantité d'apport du premier combustible recyclé incluses dans les informations sur l'apport de combustible (40).

7. Dispositif électronique (100) selon la revendication 1,
où l'un ou plusieurs processeurs (130) sont en outre configurés pour :

identifier des informations de commande, lorsqu'une entrée utilisateur correspondant aux informations de guidage est reçue par l'intermédiaire de l'UI, correspondant à l'entrée utilisateur reçue ; et

transmettre les informations de commande identifiées à un moteur de commande par l'intermédiaire de l'interface de communication (110).

8. Procédé de commande d'un dispositif électronique (100) destiné à mettre en œuvre un système de prévision et de régulation de la température, le procédé de commande comprenant :

effectuer un prétraitement, lorsque des informations de processus (50) comprenant des informations sur l'apport de combustible (40) d'un appareil de fabrication de ciment (1) sont reçues via l'interface de communication (110), des informations de processus reçues (50) ;
introduire, dans le deuxième modèle de réseau neuronal entraîné, un ensemble de données contenant i) les informations de processus prétraitées (50), ii) des premières informations de température prédite fournies par le premier modèle de réseau neuronal entraîné et iii) la température mesurée d'une première chambre de préchauffage dans l'appareil de fabrication de ciment (1) afin d'obtenir des informations d'erreur entre les premières informations de température prédite et la température mesurée de la première chambre de préchauffage, où la température mesurée de la première chambre de préchauffage est incluse dans les informations de processus prétraitées (50) ;
identifier des informations calorifiques prédites d'un premier combustible recyclé sur la base des informations d'erreur obtenues et des informations sur l'apport de combustible (40), où les informations sur le premier combustible recyclé sont incluses dans les informations sur l'apport de combustible (40) ;
mettre à jour les informations sur l'apport de combustible (40) sur la base des informations calorifiques prédites identifiées ;
introduire les informations sur l'apport de combustible actualisées (40) dans le premier modèle de réseau neuronal entraîné afin d'obtenir des deuxièmes informations de température prédite de la première chambre de préchauffage ; et
fournir une interface utilisateur, UI, comprenant des informations de guidage pour maintenir une température cible de la première chambre de préchauffage, où les informations de guidage correspondant aux deuxièmes informations de température prédite obtenues.

9. Procédé de commande selon la revendication 8, où, lorsque des informations sur une quantité d'apport correspondant à chacun des différents types de combustibles apportés, y compris le premier combustible recyclé, et les informations sur l'apport de combustible (40) comprenant des informations calorifiques correspondant à chacun des combustibles apportés sont introduites, le premier modèle de réseau neuronal entraîné est entraîné pour fournir des informations de température prédite de la première chambre de préchauffage.

10. Procédé de commande selon la revendication 8, où les informations sur l'apport de combustible (40) comprennent des informations sur une quantité d'apport correspondant à chacun des différents types de combustibles apportés, comprenant au moins l'un parmi le premier combustible recyclé, le combustible principal et le combustible auxiliaire, ainsi que des informations calorifiques correspondant à chaque quantité d'apport, et
la fourniture des informations de guidage comprend :

recevoir une valeur de température cible de la première chambre de préchauffage dans l'appareil de fabrication de ciment (1) via l'UI ;
identifier la quantité d'apport correspondant à chacun des différents types de combustibles apportés afin de garantir que la valeur de température de la première chambre de préchauffage atteigne la valeur de température cible sur la base des deuxièmes informations de température prédite obtenues et de la valeur de température cible reçue ; et
obtenir des informations de guidage correspondant à la quantité d'apport identifiée ; et
le procédé de commande comprenant en outre fournir une UI comprenant les informations de guidage obtenues.

11. Procédé de commande selon la revendication 10, où l'obtention des informations de guidage comprend en outre :

identifier des sous-informations sur l'apport de combustible dans lesquelles la quantité d'apport correspondant à chaque apport de combustible incluse dans les informations sur l'apport de combustible (40) a changé ;
introduire les sous-informations sur l'apport de combustible dans le premier modèle de réseau neuronal entraîné afin d'obtenir des deuxièmes sous-informations de température prédite ;
obtenir des informations de guidage sur le combustible apporté en utilisant les deuxièmes sous-informations de température prédite obtenues et les deuxièmes sous-informations de température prédite ; et
fournir des informations de guidage comprenant les informations de guidage obtenues.

12. Procédé de commande selon la revendication 10, où les informations de processus prétraitées (50) comprennent des informations d'historique de température de la première chambre de préchauffage et des informations d'historique du combustible apporté, et

la fourniture de l'UI comprend la fourniture d'une UI comprenant les informations d'historique de température reçues de la première chambre de préchauffage, les informations d'historique du combustible apporté et les deuxièmes informations de température prédite obtenues.

13. Procédé de commande selon la revendication 8, où l'identification des informations calorifiques comprend l'identification d'informations calorifiques prédites du premier combustible recyclé sur la base des informations d'erreur obtenues et des informations sur une quantité d'apport du premier combustible recyclé incluses dans les informations sur l'apport de combustible (40).

14. Procédé de commande selon la revendication 8, comprenant en outre :

    identifier des informations de commande, lorsqu'une entrée utilisateur correspondant aux informations de guidage est reçue via l'UI, correspondant à l'entrée utilisateur reçue ; et
    transmettre les informations de commande identifiées à un moteur de commande.

15. Support d'enregistrement non transitoire lisible par ordinateur qui stocke, lorsqu'il est exécuté par un processeur (130) d'un dispositif électronique (100) pour mettre en œuvre un système de prédiction et de contrôle de la température, des instructions informatiques qui amènent le dispositif électronique (100) à effectuer les opérations suivantes :

    effectuer un prétraitement, lorsque des informations de processus (50) comprenant des informations sur l'apport de combustible (40) d'un appareil de fabrication de ciment (1) sont reçues via l'interface de communication (110), des informations de processus reçues (50) ;
    introduire, dans le deuxième modèle de réseau neuronal entraîné, un ensemble de données contenant i) les informations de processus prétraitées (50), ii) des premières informations de température prédite fournies par le premier modèle de réseau neuronal entraîné et iii) la température mesurée d'une première chambre de préchauffage dans l'appareil de fabrication de ciment (1) afin d'obtenir des informations d'erreur entre les premières informations de température prédite et la température mesurée de la première chambre de préchauffage, où la température mesurée de la première chambre de préchauffage est incluse dans les informations de processus prétraitées (50) ;
identifier des informations calorifiques prédites d'un premier combustible recyclé sur la base des informations d'erreur obtenues et des informations sur l'apport de combustible (40), où les informations sur le premier combustible recyclé sont incluses dans les informations sur l'apport de combustible (40) ;
mettre à jour les informations sur l'apport de combustible (40) sur la base des informations calorifiques prédites identifiées ;
introduire les informations sur l'apport de combustible actualisées (40) dans le premier modèle de réseau neuronal entraîné afin d'obtenir des deuxièmes informations de température prédite de la première chambre de préchauffage ; et
fournir une interface utilisateur, UI, comprenant des informations de guidage pour maintenir une température cible de la première chambre de préchauffage, où les informations de guidage correspondant aux deuxièmes informations de température prédite obtenues.

## FIG. 1

RAW MATERIAL INPUT

AIR PATH
COOLER AIR PATH
RAW MATERIAL PATH

EXIT

TEMPERATURE OF
PREHEATING CHAMBER
MAINTAINED AT 850 °C

20

10

PYRO-ROTOR

MAIN FUEL

PREHEATING CHAMBER

AUXILIARY FUEL

CONVEYOR BELT

KILN

## FIG. 2

100

110

COMMUNICATION
INTERFACE

130

PROCESSOR

120

MEMORY

**FIG. 3**

START

↓

N ⟨ IS PROCESS INFORMATION INCLUDING FUEL INPUT INFORMATION
OF CEMENT MANUFACTURING APPARATUS RECEIVED? ⟩

↓ Y

PERFORM PREPROCESSING ON RECEIVED PROCESS INFORMATION — S320

↓

INPUT PREPROCESSED PROCESS INFORMATION INTO TRAINED SECOND NEURAL
NETWORK MODEL TO OBTAIN ERROR INFORMATION ON FIRST PREDICTED
TEMPERATURE INFORMATION OUTPUT FROM TRAINED FIRST NEURAL NETWORK
MODEL AND THE MEASURED TEMPERATURE OF FIRST PREHEATING CHAMBER IN
CEMENT MANUFACTURING APPARATUS — S330

↓

IDENTIFY PREDICTED CALORIFIC INFORMATION OF FIRST RECYCLED FUEL AMONG
INPUT FUELS BASED ON OBTAINED ERROR INFORMATION AND FUEL INPUT
INFORMATION — S340

↓

INPUT FUEL INPUT INFORMATION UPDATED BASED ON IDENTIFIED PREDICTED
CALORIFIC INFORMATION INTO TRAINED FIRST NEURAL NETWORK MODEL TO
OBTAIN SECOND PREDICTED TEMPERATURE INFORMATION OF FIRST PREHEATING
CHAMBER — S350

↓

PROVIDE GUIDANCE INFORMATION INCLUDING OBTAINED SECOND PREDICTED
TEMPERATURE INFORMATION — S360

↓

END

## FIG. 4

40

FUEL INPUT INFORMATION

41

TEMPERATURE INFORMATION OF
FIRST PREHEATING CHAMBER

⇓

FIRST NEURAL NETWORK
MODEL

400

## FIG. 5

50

PROCESS INFORMATION

51

PREDICTED
TEMPERATURE INFORMATION
OUTPUT FROM TRAINED
FIRST NEURAL NETWORK
MODEL

52

MEASURED
TEMPERATURE INFORMATION
OF FIRST PREHEATING
CHAMBER

⇓

SECOND NEURAL NETWORK
MODEL

500

**FIG. 6**

START

RECEIVE TARGET TEMPERATURE VALUE OF FIRST PREHEATING CHAMBER IN CEMENT MANUFACTURING APPARATUS — S610

IDENTIFY INPUT AMOUNT CORRESPONDING TO EACH OF DIFFERENT TYPES OF INPUT FUELS FOR TEMPERATURE VALUE OF FIRST PREHEATING CHAMBER TO REACH TARGET TEMPERATURE VALUE BASED ON OBTAINED SECOND PREDICTED TEMPERATURE INFORMATION AND RECEIVED TARGET TEMPERATURE VALUE — S620

OBTAIN GUIDANCE INFORMATION CORRESPONDING TO IDENTIFIED INPUT AMOUNT — S630

PROVIDE UI INCLUDING OBTAINED GUIDANCE INFORMATION — S640

END

**FIG. 7**

START

IDENTIFY SUB-FUEL INPUT INFORMATION IN WHICH INPUT AMOUNT CORRESPONDING TO EACH INPUT FUEL INCLUDED IN FUEL INPUT INFORMATION HAS CHANGED — S710

INPUT SUB-FUEL INPUT INFORMATION INTO TRAINED FIRST NEURAL NETWORK MODEL TO OBTAIN SECOND SUB-PREDICTED TEMPERATURE INFORMATION — S720

OBTAIN GUIDANCE INFORMATION ON INPUT FUEL USING OBTAINED SECOND PREDICTED TEMPERATURE INFORMATION AND SECOND SUB-PREDICTED TEMPERATURE INFORMATION — S730

PROVIDE GUIDE INFORMATION INCLUDING OBTAINED GUIDANCE INFORMATION — S740

END

FIG. 8A

# FIG. 8B

822

SET MIN&MAX OF AUXILIARY FUEL/RECYCLED FUEL

[ SET MIN & MAX OF AUXILIARY FUEL ARE ¡8°C AND 25°C¡ ]

AUXILIARY FUEL

MIN: | 8 |    MAX: | 25 |    | SETTING |

[ SET MIN & MAX OF RECYCLED FUEL ARE ¡2°C AND 18°C¡ ]

RECYCLED FUEL

MIN: | 2 |    MAX: | 18 |    | SETTING |

CLOSE

| Done |

823

AUXILIARY FUEL

ACTUAL VALUE

GUIDANCE VALUE

10
0
-10
-20
-30
-40
-50

12:00   00:00   12:00   00:00   12:00
Dec 8, 2022    Dec 7, 2022    Dec 6, 2022

RECYCLED FUEL

ACTUAL VALUE

GUIDANCE VALUE

20
18
16
14
12
10

12:00   00:00   12:00   00:00   12:00
Dec 8, 2022    Dec 7, 2022    Dec 6, 2022

31

## FIG. 9A

K6 KILN GUIDANCE — 910

|  | MCOAL | PCOAL | MRDF | PRDF |  |
|---|---|---|---|---|---|
| PRESENT | 8.1 | 8.1 | 5.5 | 10.1 | 901 |
| CHANGE | 0.0 | -1.6 | 0/0 | 2.0 | 902 |
| CHANGE | 8.1 | 6.5 | 5.5 | 12.1 | 903 |
| EFFECT | 0.0 | -9.7 | 0.0 | 14.6 | 904 |

## FIG. 9B

TEMPERATURE OF PREHEATING CHAMBER AFTER 5 MINUTES — 910

| PREDICTED | GUIDE PREDICTED | EFFECT |
|---|---|---|
| 882 | 887 | 5 |

— 911

**FIG. 9C**

FIRST STAGE TEMPERATURE OF K6 PREHEATING CHAMBER (°C)

ACTUAL VALUE   PREDICTED VALUE

950
900
850

15:45   16:00   16:15   16:30   16:45   17:00   17:15   17:30   17:45   18:00

Jan 25, 2023

921

| FUEL AMOUNT TREND | SET MIN&MAX OF AUXILIARY FUEL/RECYCLED FUEL |
|---|---|

923   922

[ CURRENT GUIDANCE TARGET TEMPERATURE IS 900°C ]

SET FIRST STAGE TARGET TEMPERATURE OF K6 PREHEATING CHAMBER     [          ]   SETTING

924

KILN GUIDANCE

|  | MCOAL | PCOAL | MRDF | PRDF |
|---|---|---|---|---|
| PRESENT | 8.0 | 5.1 | 6.0 | 18.2 |
| CHANGE | 0.0 | -0.1 | 0.0 | 0.3 |
| GUIDANCE VALUE | 8.0 | 5.0 | 6.0 | 18.5 |
| EFFECT | 0.0 | -0.4 | 0.0 | 0.9 |

925

TEMPERATURE OF PREHEATING CHAMBER AFTER 5 MINUTES

| PREDICTED | GUIDE PREDICTED | EFFECT |
|---|---|---|
| 902 | 902 | 0 |

926

33

# FIG. 10

START

IDENTIFY CONTROL INFORMATION CORRESPONDING TO RECEIVED USER INPUT WHEN USER INPUT CORRESPONDING TO OBTAINED GUIDANCE INFORMATION IS RECEIVED — S1010

TRANSMIT IDENTIFIED CONTROL INFORMATION TO THE CONTROL ENGINE — S1020

END

# FIG. 11

<u>100'</u>

110 COMMUNICATION INTERFACE

120 MEMORY

140 MICROPHONE

130 PROCESSOR

150 SPEAKER

160 DISPLAY

170 USER INTERFACE

180 AT LEAST ONE SENSOR

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2023274992 A1
- CN 116382371 A
- CN 114912712 A

- WO 2023067154 A1
- WO 2018038279 A1

**Non-patent literature cited in the description**

- **CHEN WEI et al.** Model Predictive Control of Outlet Temperature of Calciner Considered RDF Mixing Ratio. *2022 41 ST CHINESE CONTROL CONFERENCE (CCC), TECHNICAL COMMITTEE ON CONTROL THEORY, CHINESE ASSOCIATION OF AUTOMATION*, 25 July 2022, 2537-2543